# EUROPEAN PATENT APPLICATION

(11) **EP 2 574 667 A1**
(43) Date of publication of application: **03.04.2013**
(21) Application number: 11183652.4
(22) Date of filing: 02.10.2011
(51) Int. Cl.: C12N 9/02, C12N 15/09

(54) **Enzyme variants**

(71) Applicant: Technische Universität Graz, 8010 Graz (AT)
(72) Inventor: Straganz, Grit Daniela, 8010 Graz (AT); Konstantinovics, Cornelia Corinna, 8010 Graz (AT); Pratter, Sarah Maria, 8010 Graz (AT); Di Giuro, Cristiana Maria Loredana, 8020 Graz (AT)
(74) Representative: Sonn & Partner Patentanwälte

(57) **Abstract**

The present invention relates to a variant of an alpha-keto acid dependent enzyme catalyzing the oxidative decarboxylation of an alpha-keto acid having an amino acid sequence varying from that of a precursor alpha-keto acid dependent enzyme catalyzing the oxidative decarboxylation of an alpha-keto acid, said precursor enzyme comprising
- a motif (A) X₁X₂X₃X₄(X)ₙFGX₅X₆NX₇X₈X₉X₁₀X₁₁, wherein X₁ is serine, X₂ is an amino acid residue selected from the group consisting of threonine, lysine, isoleucine, arginine and glutamine, X₃ is valine or alanine and X₄ is an amino acid residue selected from the group consisting of valine, methionine and isoleucine, X₅ and X₆ are independently any amino acid residue, X₇ is isoleucine or phenylalanine, X₈ is an amino acid residue selected from the group consisting of lysine, glutamine, arginine and threonine, X₉ is an amino acid residue selected from the group consisting of alanine, serine and glycine, X₁₀ is leucine or isoleucine, X₁₁ is tyrosine or phenylalanine, X is any amino acid residue, and n is an integer between 100 and 150, and
- a metal ion binding motif (B) H(X₁₂)ₘH(X₁₃)ₒE, wherein X₁₂ and X₁₃ are independently any amino acid residue, m and o are independently an integer between 70 and 90,

wherein amino acid residue X₁ and/or amino acid residue X₃ within motif (A) of the variant is an amino acid residue having a molecular volume of more than 150 Å³ and/or amino acid residue X₁₁ within motif (A) of the variant has an amino acid residue having a molecular volume of less than 120 Å³.

## Description

The present invention relates to a variant of a wild-type alpha-keto acid dependent enzyme catalyzing the oxidative decarboxylation of an alpha-keto acid.

Chirality is an omnipresent concept in nature and the natural synthesis of chiral compounds via enzymatic reactions has long fascinated and inspired chemists and natural enzymes are a valuable source for enantio-specific catalysis. Often, however, these naturally evolved enzymes are not harnessed for the aspired 'non-natural' transformation. A major goal in bio-catalysis is, therefore, to control the stereo- and enantio-specificity of a biosynthetic reaction by introducing structural changes into the protein. In recent years some successes have been achieved. The majority of successful examples targets the enzyme's preference for one enantiomer over the other, (in order to resolve a racemate) or the orientation of the respective enantiomeric substrate in the active site pocket and both, a rational design based on structural information and protein evolutionary approaches, which depend on the screening of large numbers of protein-variants, have been used.

As for O₂ dependent enzymes, little is known regarding the structural basis of their enantiospecificity and a change of either enantiomer-preference or a change of enantiospecificty during the conversion of achiral substrates has never been reported and never been attempted.

S-p-hydroxy-mandelic acid synthase (S-Hms, HMS), for instance, is an exponent of an enantiospecific mononuclear nonheme-iron enzyme (MNHEE). It converts the cell metabolite p-hydroxyphenyl-pyruvic acid S-p-hydroxy-mandelic acid at high enantiospecificity (>95%) using O₂ as the sole co-substrate. The simplicity of the reaction that uses two bulk chemicals to produce the valuable chiral acid is intriguing, all the more, as for chemical synthesis the use of dioxygen for controlled oxidations is still a great challenge and the reproduction of such an enantio-specific reaction is, so far, out of reach. The apparent simplicity of the reaction is in stark contrast to the enzymatic mechanism employed:
In line with the principle mechanistic rationale of α-ketoacid dependent dioxygenases the reaction pathway of S-HMS proceeds as follows. Ligation of the α-keto-acid moiety to the ferrous iron center primes the metal center for the reduction of O₂, which is concomitantly decarboxylated. Homolytic cleavage of the O-O bond of the resulting peroxidate intermediate yields p-hydroxy-phenylacetate and the high valent iron-oxo intermediate Fe(IV)=O²⁻. This strong oxidizing agent then abstracts a H-atom from the alpha carbon of p-hydroxy-phenylacetic acid and subsequently performs the hydroxylation of the radical-intermediate. This last step ultimately determines the enantiospecificity of the reaction and in an achiral, non-restricting environment R- and S-mandelate derivatives would be formed from the planar radical-intermediate at equal ratios. Yet, the actual enantiospecificity for mandelate synthesis from phenyl pyruvate of HMS is >99% (for S-mandelate)(see Example 1).

It is an object of the present invention to provide means and methods to produce alpha-hydroxy acids from first alpha-keto-acids using enzymes or whole cells producing such enzymes.

Therefore the present invention relates to a variant of an alpha-keto acid dependent enzyme catalyzing the oxidative decarboxylation of an alpha-keto acid having an amino acid sequence varying from that of a precursor alpha-keto acid dependent enzyme catalyzing the oxidative decarboxylation of an alpha-keto acid, said precursor enzyme comprising
- a motif (A) X₁X₂X₃X₄(X)ₙFGX₅X₆NX₇X₈X₉X₁₀X₁₁, wherein X₁ is serine, X₂ is an amino acid residue selected from the group consisting of threonine, lysine, isoleucine, arginine and glutamine, X₃ is valine or alanine and X₄ is an amino acid residue selected from the group consisting of valine, methionine and isoleucine, X₅ and X₆ are independently any amino acid residue, X₇ is isoleucine or phenylalanine, X₈ is an amino acid residue selected from the group consisting of lysine, glutamine, arginine and threonine, X₉ is an amino acid residue selected from the group consisting of alanine, serine and glycine, X₁₀ is leucine or isoleucine, X₁₁ is tyrosine or phenylalanine, X is any amino acid residue, and n is an integer between 100 and 150, and
- a metal ion binding motif (B) H(X₁₂)ₘH(X₁₃)ₒE, wherein X₁₂ and X₁₃ are independently any amino acid residue, m and o are independently an integer between 70 and 90,
wherein amino acid residue X₁ and/or amino acid residue X₃ within motif (A) of the variant is an amino acid residue having a volume of more than 150 Å³ and/or amino acid residue X₁₁ within motif (A) of the variant has an amino acid residue having a volume of less than 120 Å³_{.}

It turned surprisingly out that the exchange of one or more, preferably of two or more, more preferably of three or more, specific amino acid residues within motif (A) of an alpha-keto acid dependent enzyme catalyzing the oxidative decarboxylation of an alpha-keto acid with amino acid residues having a defined volume results in a variant which is able to produce a specific enantiomer of an alpha-keto acid at a higher ratio compared to the unmodified precursor. In a particularly preferred embodiment of the present invention the variant and/or its precursor enzyme are able to decarboxylate an alpha-keto acid comprising an aromatic ring, preferably a compound according to formula (II) (see below). A person skilled in the art is able to identify respective enzymes.

Motif (A) as defined above is part of at least one, preferably two, binding pockets binding to at least one product which is obtained when incubating the enzyme of the present invention with a substrate, (whereby the preferred binding of one product enantiomer reflects the preferred formation of that enantiomer during catalysis). If the product obtained by incubating a substrate with the enzyme of the present invention is an enantiomer one binding pocket may bind the S-enantiomer and/or one binding pocket may bind to the R-enantiomer. For instance, wild-type hydroxymandelate synthase (HMS; EC 1.13.11.46) comprising a motif (A) as defined above catalyzes the decarboxylation of 4-hydroxyphenylpyruvate to S-mandelate and to a much lower extend to R-mandelate. One binding pocket of HMS binds the S-enantiomer and a second pocket binds or potentially binds the R-enantiomer of mandelate. In case of 4-hydroxyphenylpyruvate dioxygenase (HPPD; EC 1.13.11.27), for instance, the enzyme can be modified as described in O'Hare HM et al. (FEBS Letters 580(2006):3445-3450) to be able to convert 4-hydroxyphenylpyruvate into S- and/or R-mandelate, whereby O'Hare HM et al. created only enzymes which were able to produce S-mandelate. Also such a variant of HPPD comprises motif (A) and is thus able to bind the respective S- and R-enantiomer. If one (the S-mandelate specific) binding of the alpha-keto acid dependent enzyme is modified in a way to hinder that a specific enantiomer binds thereto the respective enantiomer is formed to a lower extent compared to the precursor alpha-keto acid dependent enzyme. For this reason amino acid residue X₁ and/or X₃ of the precursor alpha-keto acid dependent enzyme (being part of the binding pocket of an S-enantiomer of an alpha-keto acid) is exchanged with an amino acid residue which has a larger volume than the respective amino acid residues present in the precursor enzyme. Particularly preferred the amino acid residue X₁ and/or amino acid residue X₃ is selected from the group consisting of arginine, histidine, isoleucine, leucine, lysine, methionine, phenylalanine, tryptophan and tyrosine, preferably selected from the group consisting of phenylalanine, tryptophan and methionine, more preferably phenylalanine or methionine. Amino acid residue X₁₁ within motif (A) being part of the (hypothetical) binding pocket of an R-enantiomer is exchanged with an amino acid residue having a molecular volume which is lower than the molecular volume of tyrosine or phenylalanine, preferably lower than 120 Å³. This amino acid exchange increases the pocket in which the R-enantiomer binds thus leading to an increased formation of the respective enantiomer compared to the precursor alpha-keto acid dependent enzyme.

The term "amino acid residue having a molecular volume" refers to the volume of an amino acid residue Å³ as defined for instance in Bondi AJ (J Phys Chem 68(1964):441-451).

The amino acid molecular volumes are indicated in the following table (see also Perkins SJ Eur J Biochem 157(1986):169-180) :

| **Amino acid** | **Abbreviations** | | **Volume (Å³)** |
|---|---|---|---|
| Alanine | Ala | A | 87.8 |
| Arginine | Arg | R | 188.2 |
| Asparagine | Asn | N | 120.1 |
| Aspartic acid | Asp | D | 115.4 |
| Cysteine | Cys | C | 105.4 |
| Glutamine | Gln | Q | 145.1 |
| Glutamic Acid | Glu | E | 140.9 |
| Glycine | Gly | G | 59.9 |
| Histidine | His | H | 156.3 |
| Isoleucine | Ile | I | 166.1 |
| Leucine | Leu | L | 168.0 |
| Lysine | Lys | K | 172.7 |
| Methionine | Met | M | 165.2 |
| Phenylalanine | Phe | F | 189.7 |
| Proline | Pro | P | 123.3 |
| Serine | Ser | S | 91.7 |
| Threonine | Thr | T | 118.3 |
| Tryptophan | Trp | W | 227.9 |
| Tyrosine | Tyr | Y | 191.2 |
| Valine | Val | V | 138.8 |

| | | | |
|---|---|---|---|
| Å³ is Angström ³ | | | |

An "alpha-keto acid dependent enzyme catalyzing the oxidative decarboxylation of an alpha-keto acid" as used herein refers to enzymes which are able to decarboxylate and optionally to (subsequently) hydroxylate alpha-keto acids. Examples of such enzymes are 4-hydroxyphenylpyruvate:oxygen oxidoreductases such as 4-hydroxyphenylpyruvate dioxygenase and 4-hydroxymandelate synthase.

Within the scope of the present invention are also functional fragments of the variant of the present invention which still retain the functional properties of the full-length enzyme.

A "precursor" of the variant of the present invention is the enzyme from which the variant is derived from. The "precursor" enzyme may be, for instance, a wildtype enzyme of any source provided that the enzyme is able to catalyze the oxidative decarboxylation of an (preferably aromatic) alpha-keto acid (e.g. alpha keto acids with an aromatic substituent) or catalyze the oxidative decarboxylation and subsequent oxygenation/hydroxylation of the substrate

In a particular preferred embodiment of the present invention amino acid residue X₁ and/or amino acid residue X₃ within motif (A) of the variant is an amino acid residue having a volume of more than 150 Å³, preferably more than 160 Å³. In addition thereto or alternatively amino acid residue X₁₁ within motif (A) of the variant may have an amino acid residue having a molecular volume of less than 120 Å³, preferably less than 100 Å³.

Also encompassed in the present invention are variants of alpha-keto acid dependent enzymes catalyzing the oxidative decarboxylation of an alpha-keto acid which comprise motifs (A) and (B) and which are derived from precursor enzymes which are at least 80%, preferably at least 90%, more preferably at least 95%, even more preferably at least 98%, most preferably at least 99%, identical to SEQ ID No. 1
SEQ ID No. 1:

MLPPFPFLHWRAAMPPSDIAYAELYVADDREASGFLVDSLGFVPLAVAGPATGTHDRRSTV
LRSGEVTLVVTQALAPDTPVARYVERHGDSIADLAFGCDDVRSCFDRAVLAGAEALQAPTPSHRAG
QDAWFATVSGFGDIRHTLVPAADGDGAGLLPPDRDWALLPAATGRTGPRPLLDHVAVCLESGTLRS
TAEFYEAAFDMPYYSSEYIEVGEQAMDSIVVRNAGGGITFTLIEPDDTRVPGQIDQFLSAHDGPGV
QHLAFLVDDIVGSVRSLGDRGVAFLRTPGAYYDLLTERVGAMADAIEDLRETNVLADRDEWGYLLQ
IFTRSPYPRGTLFYEYIQRNGARGFGSSNIKALYEAVEREREVAGR

As used herein, the term "sequence identity" and "identical" refers to two or more sequences or subsequences that have at least the aforementioned amino acid residue identity, when compared and aligned for maximum correspondence, as measured using the following sequence comparison algorithm.

Two sequences (amino acid) can be compared over their full-length (e.g., the length of the shorter of the two, if they are of substantially different lengths) or over a subsequence such at least about 10, about 20, about 30, about 50 or about 100 contiguous amino acid residues. For sequence comparison, typically one sequence acts as a reference sequence, to which test sequences are compared. In the present case SEQ ID No. 1 is used as such a reference sequence. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters. Optimal alignment of sequences for comparison can be conducted, e.g., by the local homology algorithm of Smith &; Waterman, Adv. Appl. Math. 2:482 (1981), by the homology alignment algorithm of Needleman &; Wunsch, J. Mol. Biol. 48:443 (1970), by the search for similarity method of Pearson &; Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., Current Protocols In Molecular Biology, Greene Publishing and Wiley-Interscience, New York (supplemented through 1999). When using any of the aforementioned algorithms, the default parameters for "Window" length, gap penalty, etc., are used. One example of algorithm that is suitable and most preferred for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (NCBI). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits act as seeds for initiating searches to find longer HSPs containing them. The word hits are then extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff &; Henikoff, Proc. Natl. Acad. Sci. USA 89:10915 (1989)) alignments (B) of 50, expectation (E) of 10, M=5, N-4, and a comparison of both strands. In addition to calculating percent sequence identity, the BLAST algorithm also performs a statistical analysis of the similarity between two sequences (see, e.g., Karlin &; Altschul, Proc. Natl. Acad. Sci. USA 90:5873-5787 (1993)).

According to a preferred embodiment of the present invention amino acid residue X₁₁ is selected from the group consisting of alanine, asparagine, aspartic acid, cysteine, glycine, proline, serine and threonine, preferably serine, glycine or alanine, more preferably alanine.

According to a particularly preferred embodiment of the present invention the variant of the present invention may comprise the following substitutions within motif (A) compared to the precursor enzyme:

| X₁ | X₃ | X₁₁ | X₁ | X₃ | X₁₁ | X₁ | X₃ | X₁₁ |
|---|---|---|---|---|---|---|---|---|
| R | - | - | K | H | D | R | H | T |
| H | - | - | M | H | D | H | H | T |
| I | - | - | F | H | D | I | H | T |
| L | - | - | W | H | D | L | H | T |
| K | - | - | Y | H | D | K | H | T |
| M | - | - | R | I | D | M | H | T |
| F | - | - | H | I | D | F | H | T |
| W | - | - | I | I | D | W | H | T |
| Y | - | - | L | I | D | Y | H | T |
| - | R | - | K | I | D | R | I | T |
| - | H | - | M | I | D | H | I | T |
| - | I | - | F | I | D | I | I | T |
| - | L | - | W | I | D | L | I | T |
| - | K | - | Y | I | D | K | I | T |
| - | M | - | R | L | D | M | I | T |
| - | F | - | H | L | D | F | I | T |
| - | W | - | I | L | D | W | I | T |
| - | Y | - | L | L | D | Y | I | T |
| - | - | A | K | L | D | R | L | T |
| - | - | N | M | L | D | H | L | T |
| - | - | D | F | L | D | I | L | T |
| - | - | C | W | L | D | L | L | T |
| - | - | G | Y | L | D | K | L | T |
| - | - | P | R | K | D | M | L | T |
| - | - | S | H | K | D | F | L | T |
| - | - | T | I | K | D | W | L | T |
| R | R | - | L | K | D | Y | L | T |
| H | R | - | K | K | D | R | K | T |
| I | R | - | M | K | D | H | K | T |
| L | R | - | F | K | D | I | K | T |
| K | R | - | W | K | D | L | K | T |
| M | R | - | Y | K | D | K | K | T |
| F | R | - | R | M | D | M | K | T |
| W | R | - | H | M | D | F | K | T |
| Y | R | - | I | M | D | W | K | T |
| R | H | - | L | M | D | Y | K | T |
| H | H | - | K | M | D | R | M | T |
| I | H | - | M | M | D | H | M | T |
| L | H | - | F | M | D | I | M | T |
| K | H | - | W | M | D | L | M | T |
| M | H | - | Y | M | D | K | M | T |
| F | H | - | R | F | D | M | M | T |
| W | H | - | H | F | D | F | M | T |
| Y | H | - | I | F | D | W | M | T |
| R | I | - | L | F | D | Y | M | T |
| H | I | - | K | F | D | R | F | T |
| I | I | - | M | F | D | H | F | T |
| L | I | - | F | F | D | I | F | T |
| K | I | - | W | F | D | L | F | T |
| M | I | - | Y | F | D | K | F | T |
| F | I | - | R | W | D | M | F | T |
| W | I | - | H | W | D | F | F | T |
| Y | I | - | I | W | D | W | F | T |
| R | L | - | L | W | D | Y | F | T |
| H | L | - | K | W | D | R | W | T |
| I | L | - | M | W | D | H | W | T |
| L | L | - | F | W | D | I | W | T |
| K | L | - | W | W | D | L | W | T |
| M | L | - | Y | W | D | K | W | T |
| F | L | - | R | Y | D | M | W | T |
| W | L | - | H | Y | D | F | W | T |
| Y | L | - | I | Y | D | W | W | T |
| R | K | - | L | Y | D | Y | W | T |
| H | K | - | K | Y | D | R | Y | T |
| I | K | - | M | Y | D | H | Y | T |
| L | K | - | F | Y | D | I | Y | T |
| K | K | - | W | Y | D | L | Y | T |
| M | K | - | Y | Y | D | K | Y | T |
| F | K | - | R | R | C | M | Y | T |
| W | K | - | H | R | C | F | Y | T |
| Y | K | - | I | R | C | W | Y | T |
| R | M | - | L | R | C | Y | Y | T |
| H | M | - | K | R | C | - | R | A |
| I | M | - | M | R | C | - | H | A |
| L | M | - | F | R | C | - | I | A |
| K | M | - | W | R | C | - | L | A |
| M | M | - | Y | R | C | - | K | A |
| F | M | - | R | H | C | - | M | A |
| W | M | - | H | H | C | - | F | A |
| Y | M | - | I | H | C | - | W | A |
| R | F | - | L | H | C | - | Y | A |
| H | F | - | K | H | C | - | R | N |
| I | F | - | M | H | C | - | H | N |
| L | F | - | F | H | C | - | I | N |
| K | F | - | W | H | C | - | L | N |
| M | F | - | Y | H | C | - | K | N |
| F | F | - | R | I | C | - | M | N |
| W | F | - | H | I | C | - | F | N |
| Y | F | - | I | I | C | - | W | N |
| R | W | - | L | I | C | - | Y | N |
| H | W | - | K | I | C | - | R | D |
| I | W | - | M | I | C | - | H | D |
| L | W | - | F | I | C | - | I | D |
| K | W | - | W | I | C | - | L | D |
| M | W | - | Y | I | C | - | K | D |
| F | W | - | R | L | C | - | M | D |
| W | W | - | H | L | C | - | F | D |
| Y | W | - | I | L | C | - | W | D |
| R | Y | - | L | L | C | - | Y | D |
| H | Y | - | K | L | C | - | R | C |
| I | Y | - | M | L | C | - | H | C |
| L | Y | - | F | L | C | - | I | C |
| K | Y | - | W | L | C | - | L | C |
| M | Y | - | Y | L | C | - | K | C |
| F | Y | - | R | K | C | - | M | C |
| W | Y | - | H | K | C | - | F | C |
| Y | Y | - | I | K | C | - | W | C |
| R | R | A | L | K | C | - | Y | C |
| H | R | A | K | K | C | - | R | G |
| I | R | A | M | K | C | - | H | G |
| L | R | A | F | K | C | - | I | G |
| K | R | A | W | K | C | - | L | G |
| M | R | A | Y | K | C | - | K | G |
| F | R | A | R | M | C | - | M | G |
| W | R | A | H | M | C | - | F | G |
| Y | R | A | I | M | C | - | W | G |
| R | H | A | L | M | C | - | Y | G |
| H | H | A | K | M | C | - | R | P |
| I | H | A | M | M | C | - | H | P |
| L | H | A | F | M | C | - | I | P |
| K | H | A | W | M | C | - | L | P |
| M | H | A | Y | M | C | - | K | P |
| F | H | A | R | F | C | - | M | P |
| W | H | A | H | F | C | - | F | P |
| Y | H | A | I | F | C | - | W | P |
| R | I | A | L | F | C | - | Y | P |
| H | I | A | K | F | C | - | R | S |
| I | I | A | M | F | C | - | H | S |
| L | I | A | F | F | C | - | I | S |
| K | I | A | W | F | C | - | L | S |
| M | I | A | Y | F | C | - | K | S |
| F | I | A | R | W | C | - | M | S |
| W | I | A | H | W | C | - | F | S |
| Y | I | A | I | W | C | - | w | S |
| R | L | A | L | W | C | - | Y | S |
| H | L | A | K | W | C | - | R | T |
| I | L | A | M | w | C | - | H | T |
| L | L | A | F | W | C | - | I | T |
| K | L | A | W | W | C | - | L | T |
| M | L | A | Y | W | C | - | K | T |
| F | L | A | R | Y | C | - | M | T |
| W | L | A | H | Y | C | - | F | T |
| Y | L | A | I | Y | C | - | W | T |
| R | K | A | L | Y | C | - | Y | T |
| H | K | A | K | Y | C | R | - | A |
| I | K | A | M | Y | C | H | - | A |
| L | K | A | F | Y | C | I | - | A |
| K | K | A | W | Y | C | L | - | A |
| M | K | A | Y | Y | C | K | - | A |
| F | K | A | R | R | G | M | - | A |
| W | K | A | H | R | G | F | - | A |
| Y | K | A | I | R | G | W | - | A |
| R | M | A | L | R | G | Y | - | A |
| H | M | A | K | R | G | R | - | N |
| I | M | A | M | R | G | H | - | N |
| L | M | A | F | R | G | I | - | N |
| K | M | A | W | R | G | L | - | N |
| M | M | A | Y | R | G | K | - | N |
| F | M | A | R | H | G | M | - | N |
| W | M | A | H | H | G | F | - | N |
| Y | M | A | I | H | G | W | - | N |
| R | F | A | L | H | G | Y | - | N |
| H | F | A | K | H | G | R | - | D |
| I | F | A | M | H | G | H | - | D |
| L | F | A | F | H | G | I | - | D |
| K | F | A | W | H | G | L | - | D |
| M | F | A | Y | H | G | K | - | D |
| F | F | A | R | I | G | M | - | D |
| W | F | A | H | I | G | F | - | D |
| Y | F | A | I | I | G | W | - | D |
| R | W | A | L | I | G | Y | - | D |
| H | W | A | K | I | G | R | - | C |
| I | W | A | M | I | G | H | - | C |
| L | W | A | F | I | G | I | - | C |
| K | W | A | W | I | G | L | - | C |
| M | W | A | Y | I | G | K | - | C |
| F | W | A | R | L | G | M | - | C |
| W | W | A | H | L | G | F | - | C |
| Y | W | A | I | L | G | W | - | C |
| R | Y | A | L | L | G | Y | - | C |
| H | Y | A | K | L | G | R | - | G |
| I | Y | A | M | L | G | H | - | G |
| L | Y | A | F | L | G | I | - | G |
| K | Y | A | W | L | G | L | - | G |
| M | Y | A | Y | L | G | K | - | G |
| F | Y | A | R | K | G | M | - | G |
| W | Y | A | H | K | G | F | - | G |
| Y | Y | A | I | K | G | W | - | G |
| R | R | N | L | K | G | Y | - | G |
| H | R | N | K | K | G | R | - | P |
| I | R | N | M | K | G | H | - | P |
| L | R | N | F | K | G | I | - | P |
| K | R | N | W | K | G | L | - | P |
| M | R | N | Y | K | G | K | - | P |
| F | R | N | R | M | G | M | - | P |
| W | R | N | H | M | G | F | - | P |
| Y | R | N | I | M | G | W | - | P |
| R | H | N | L | M | G | Y | - | P |
| H | H | N | K | M | G | R | - | S |
| I | H | N | M | M | G | H | - | S |
| L | H | N | F | M | G | I | - | S |
| K | H | N | W | M | G | L | - | S |
| M | H | N | Y | M | G | K | - | S |
| F | H | N | R | F | G | M | - | S |
| W | H | N | H | F | G | F | - | S |
| Y | H | N | I | F | G | W | - | S |
| R | I | N | L | F | G | Y | - | S |
| H | I | N | K | F | G | R | - | T |
| I | I | N | M | F | G | H | - | T |
| L | I | N | F | F | G | I | - | T |
| K | I | N | W | F | G | L | - | T |
| M | I | N | Y | F | G | K | - | T |
| F | I | N | R | W | G | M | - | T |
| W | I | N | H | W | G | F | - | T |
| Y | I | N | I | W | G | W | - | T |
| R | L | N | L | W | G | Y | - | T |
| H | L | N | K | W | G | W | M | P |
| I | L | N | M | W | G | Y | M | P |
| L | L | N | F | W | G | R | F | P |
| K | L | N | W | W | G | H | F | P |
| M | L | N | Y | W | G | I | F | P |
| F | L | N | R | Y | G | L | F | P |
| W | L | N | H | Y | G | K | F | P |
| Y | L | N | I | Y | G | M | F | P |
| R | K | N | L | Y | G | F | F | P |
| H | K | N | K | Y | G | W | F | P |
| I | K | N | M | Y | G | Y | F | P |
| L | K | N | F | Y | G | R | W | P |
| K | K | N | W | Y | G | H | W | P |
| M | K | N | Y | Y | G | I | W | P |
| F | K | N | R | R | P | L | W | P |
| W | K | N | H | R | P | K | W | P |
| Y | K | N | I | R | P | M | W | P |
| R | M | N | L | R | P | F | W | P |
| H | M | N | K | R | P | W | W | P |
| I | M | N | M | R | P | Y | W | P |
| L | M | N | F | R | P | R | Y | P |
| K | M | N | W | R | P | H | Y | P |
| M | M | N | Y | R | P | I | Y | P |
| F | M | N | R | H | P | L | Y | P |
| W | M | N | H | H | P | K | Y | P |
| Y | M | N | I | H | P | M | Y | P |
| R | F | N | L | H | P | F | Y | P |
| H | F | N | K | H | P | W | Y | P |
| I | F | N | M | H | P | Y | Y | P |
| L | F | N | F | H | P | R | R | S |
| K | F | N | W | H | P | H | R | S |
| M | F | N | Y | H | P | I | R | S |
| F | F | N | R | I | P | L | R | S |
| W | F | N | H | I | P | K | R | S |
| Y | F | N | I | I | P | M | R | S |
| R | W | N | L | I | P | F | R | S |
| H | W | N | K | I | P | W | R | S |
| I | W | N | M | I | P | Y | R | S |
| L | W | N | F | I | P | R | H | S |
| K | W | N | W | I | P | H | H | S |
| M | W | N | Y | I | P | I | H | S |
| F | W | N | R | L | P | L | H | S |
| W | W | N | H | L | P | K | H | S |
| Y | W | N | I | L | P | M | H | S |
| R | Y | N | L | L | P | F | H | S |
| H | Y | N | K | L | P | W | H | S |
| I | Y | N | M | L | P | Y | H | S |
| L | Y | N | F | L | P | R | I | S |
| K | Y | N | W | L | P | H | I | S |
| M | Y | N | Y | L | P | I | I | S |
| F | Y | N | R | K | P | L | I | S |
| W | Y | N | H | K | P | K | I | S |
| Y | Y | N | I | K | P | M | I | S |
| R | R | D | L | K | P | F | I | S |
| H | R | D | K | K | P | W | I | S |
| I | R | D | M | K | P | Y | I | S |
| L | R | D | F | K | P | R | L | S |
| K | R | D | W | K | P | H | L | S |
| M | R | D | Y | K | P | I | L | S |
| F | R | D | R | M | P | L | L | S |
| W | R | D | H | M | P | K | L | S |
| Y | R | D | I | M | P | M | L | S |
| R | H | D | L | M | P | F | L | S |
| H | H | D | K | M | P | W | L | S |
| I | H | D | M | M | P | Y | L | S |
| L | H | D | F | M | P | R | K | S |
| M | W | S | W | M | S | H | K | S |
| F | W | S | Y | M | S | I | K | S |
| W | W | S | R | F | S | L | K | S |
| Y | W | S | H | F | S | K | K | S |
| R | Y | S | I | F | S | M | K | S |
| H | Y | S | L | F | S | F | K | S |
| I | Y | S | K | F | S | W | K | S |
| L | Y | S | M | F | S | Y | K | S |
| K | Y | S | F | F | S | R | M | S |
| M | Y | S | W | F | S | H | M | S |
| F | Y | S | Y | F | S | I | M | S |
| W | Y | S | R | W | S | L | M | S |
| Y | Y | S | H | W | S | K | M | S |
| R | R | T | I | W | S | M | M | S |
| H | R | T | L | W | S | F | M | S |
| I | R | T | K | W | S | L | R | T |
| R | H | T | W | R | T | K | R | T |
| F | R | T | Y | R | T | M | R | T |

According to a particularly preferred embodiment of the present invention the variant may comprise the following amino acid residues within motif (A):

| **X₁** | **X₃** | **X₁₁** |
|---|---|---|
| M | - | - |
| - | F | - |
| - | M | - |
| - | - | A |
| - | F | A |
| M | - | A |
| M | M | A |
| M | F | A |

The most preferred variants of the present invention comprise the following amino acid residues within motif (A):

| **X₁** | **X₃** | **X₁₁** |
|---|---|---|
| M | - | - |
| - | F | - |
| - | F | A |
| M | - | A |
| M | M | A |
| M | F | A |

In a particularly preferred embodiment of the present invention the precursor enzyme is 4-hydroxymandelate synthase of Streptomyces coelicolor A3(2) (SEQ ID No. 1) and the respective most preferred variants thereof have the following amino acid sequences:
SEQ ID No. 2 (V223F; X₃ is F): 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDGAGLLPPDRDWALLPAATGRTGPRPLLDHVAVCLESGTLRSTAEFY
EAAFDMPYYSSEYIEVGEQAMDSIFVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALYEAVEREREVAGR
SEQ ID No. 3 (V223W; X₃ is W): 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDGAGLLPPDRDWALLPAATGRTGPRPLLDHVAVCLESGTLRSTAEFY
EAAFDMPYYSSEYIEVGEQAMDSIWVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALYEAVEREREVAGR
SEQ ID No. 4 (V223M; X₃ is M): 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDSIMVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALYEAVEREREVAGR
SEQ ID No. 5 (Y359A; X₁₁ is A) : 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDSIVVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALAEAVEREREVAGR
SEQ ID No. 6 (S221M; X₁ is M): 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDMIVVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALYEAVEREREVAGR
SEQ ID No. 7 (V223F_Y359A; X₃ is F and X₁₁ is A) : 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDSIFVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALAEAVEREREVAGR
SEQ ID No. 8 (S221M_Y359A; X₁ is M and X₁₁ is A) : 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDMIVVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALAEAVEREREVAGR
SEQ ID No. 9 (S221M_V223F_Y359A; X₁ is M, X₃ is F and X₁₁ is A) : 
MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDMIFVRNAGGGITFTLIEPDDTRVPGQIDQFLSAHDGPGVQHLAF
 
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALAEAVEREREVAGR
SEQ ID No. 10 (Y359A_S221M_V223M; X₁ is M, X₃ is M and X₁₁ is A) :

MLPPFPFLHWRAAMPPSDIAYAELYVADDREASGFLVDSLGFVPLAVAGPATGTHDRRSTVLRSGE
VTLVVTQALAPDTPVARYVERHGDSIADLAFGCDDVRSCFDRAVLAGAEALQAPTPSHRAGQDAWF
ATVSGFGDIRHTLVPAADGDAGLLPPDRDWALLPAATGRTGPRPLLDHVAVCLESGTLRSTAEFY
EAAFDMPYYSSEYIEVGEQAMDMIMVRNAGGGITFTLIEPDDTRVPGQIDQFLSAHDGPGVQHLAF
LVDDIVGSVRSLGDRGVAFLRTPGAYYDLLTERVGAMADAIEDLRETNVLADRDEWGYLLQIFTRS
PYPRGTLFYEYIQRNGARGFGSSNIKALAEAVEREREVAGR

Between the two active sites of motif (A) of the alpha-keto acid dependent enzyme catalyzing the oxidative decarboxylation of an alpha-keto acid of the present invention a certain number of amino acid residues depending on the specific precursor enzyme can be found. Hence, n is an integer between 100 and 150, preferably between 110 and 140, more preferably between 115 and 130, even more preferably between 120 and 125.

Alpha-keto dependent oxygenases which require the presence of a metal, preferably iron, ion for their activity comprise a 2-His-1-carboxylate facial triad that localises the active site metal ion through direct coordination. Typically these oxygenases can be categorised into 3 groups based on the spacing of these 3 lichens in the primary structure of the enzyme. However, the most preferred alpha-keto acid dependent enzyme of the present invention comprise a metal ion binding motif (B) H(X₁₂)ₘH(X₁₂)ₒE, wherein X₁₂ is any amino acid residue, m and o are independently an integer between 70 and 90, preferably between 75 and 85, more preferably between 78 and 82, in particular 80 (see e.g. Moran GR Arch Biochem Biophys 433(2005):117-128).

In a particular preferred embodiment of the present invention X₈ is lysine or glutamine and/or X₁₀ is leucine.

In a preferred embodiment of the present invention the precursor alpha-keto acid dependent enzyme catalyzing the oxidative decarboxylation of an alpha-keto acid is a hydroxyphenylpyruvate dioxygenase (EC 1.13.11.27) or a hydroxylmandelate synthase (1.13.11.46).

The precursor alpha-keto acid dependent enzyme catalyzing the oxidative decarboxylation of an alpha-keto acid is preferably derived or obtained from an organism selected from the group consisting of Streptomyces coelicolor A3(2),Amycolatopsis orientalis, Amycolatopsis balhimycina, Streptomyces toyocaensis, Streptomyces lavendulae, Nonomuraea sp. ATCC 39727, Nocardia uniformis subsp. Tsuyamanensis, Actinosynnema mirum DSM 43827, Streptosporangium roseum DSM 43021, Actinoplanes teichomyceticus, Salinispora tropica CNB-440, Streptomyces bingchenggensis BCW-1, Kribbella flavida DSM 17836, Micromonospora aurantiaca ATCC 27029, Micromonospora sp. L5, Streptomyces lividans TK24, Catenulispora acidiphila DSM 44928, Streptomyces fungicidicus, Catenulispora acidiphila DSM 44928 Salinispora arenicola CNS-205, Salinispora tropica CNB-440, Micromonospora sp. ATCC 39149, Verrucosispora maris AB-18-032 and, Streptomyces violaceusniger Tu 4113.

Another aspect of the present invention relates to a nucleic acid molecule encoding a variant of a wild-type alpha-keto acid dependent enzyme catalyzing the oxidative decarboxylation of an alpha-keto acid according to the present invention.

The nucleic acid sequences encoding the variant or its precursor enzyme can be obtained by sequencing the respective genes present in the organisms which are able to produce the precursor enzyme. Of course every nucleic acid sequence encoding the variant of the present invention or its precursor can be used.

The nucleic acid sequence of the present invention may also be codon optimized or harmonized depending on the host cell in which the variant of the present invention or its precursor enzyme are expressed or overexpressed.

Yet another aspect of the present invention relates to a vector comprising a nucleic acid molecule as defined above.

The present invention provides expression vectors and cloning vehicles comprising nucleic acids of the invention, e.g., sequences encoding the phospholipases of the invention. Expression vectors and cloning vehicles of the invention can comprise viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), P1-based artificial chromosomes, yeast plasmids, yeast artificial chromosomes, and any other vectors specific for specific host cells of interest. Vectors of the invention can include chromosomal, non-chromosomal and synthetic DNA sequences. Large numbers of suitable vectors are known to those of skill in the art, and are commercially available. Exemplary vectors are include: bacterial: pASK (IBA USA), pQE vectors (Qiagen), pBluescript plasmids, pNH vectors, (lambda-ZAP vectors (Stratagene); ptrc99a, pKK223-3, pDR540, pRIT2T (Pharmacia); Eukaryotic: pXT1, pSG5 (Stratagene), pSVK3, pBPV, pMSG, pSVLSV40 (Pharmacia). However, any other plasmid or other vector may be used so long as they are replicable and viable in the host.

The expression vector may comprise a promoter, a ribosome-binding site for translation initiation and a transcription terminator. The vector may also include appropriate sequences for amplifying expression. Mammalian expression vectors can comprise an origin of replication, any necessary ribosome binding sites, a polyadenylation site, splice donor and acceptor sites, transcriptional termination sequences, and 5' flanking non-transcribed sequences.

In one aspect, the expression vectors contain one or more selectable marker genes to permit selection of host cells containing the vector. Such selectable markers include genes encoding dihydrofolate reductase or genes conferring neomycin resistance for eukaryotic cell culture, genes conferring tetracycline or ampicillin resistance in E. coli and the S. cerevisiae TRP1 gene. Promoter regions can be selected from any desired gene using chloramphenicol transferase (CAT) vectors or other vectors with selectable markers.

A nucleic acid sequence may be inserted into a vector by a variety of procedures. In general, the nucleic acid sequence is ligated to the desired position in the vector following digestion of the insert and the vector with appropriate restriction endonucleases. A variety of cloning techniques are known in the art.

The vector may be in the form of a plasmid, a viral particle, or a phage. Other vectors include chromosomal, non-chromosomal and synthetic DNA sequences, derivatives of SV40; bacterial plasmids, phage DNA, baculovirus, yeast plasmids, vectors derived from combinations of plasmids and phage DNA, viral DNA such as vaccinia, adenovirus, fowl pox virus, and pseudorabies.

Particular bacterial vectors which may be used include the commercially available plasmids comprising genetic elements of the well known cloning vector pBR322 (ATCC 37017), pKK223-3 (Pharmacia Fine Chemicals, Sweden), GEM1 (Promega Biotec, USA) pQE70, pQE60, pQE-9 (Qiagen), pD10, psiX174 pBluescript II KS, pNH8A, pNH16a, pNH18A, pNH46A (Stratagene), ptrc99a, pKK223-3, pKK233-3, pDR540, pRIT5 (Pharmacia), pKK232-8 and pCM7. Particular eukaryotic vectors include pSV2CAT, pOG44, pXT1, pSG (Stratagene) pSVK3, pBPV, pMSG, and pSVL (Pharmacia). However, any other vector may be used as long as it is replicable and viable in the host cell.

In order to facilitate the purification of a variant of the present invention it is preferred to fuse to the variant a Tag which is able to bind to a binding partner being bound, for instance, to a solid support which can be packed into a column. Respective means and methods are known in the art.

Another aspect of the present invention relates to a host cell comprising a nucleic acid molecule or a vector according to the present invention.

The invention also provides a transformed cell comprising a nucleic acid sequence or a vector as described above. The host cell may be any of the host cells familiar to those skilled in the art, including prokaryotic cells, eukaryotic cells, such as bacterial cells, fungal cells, yeast cells, mammalian cells, insect cells, or plant cells. Enzymes of the invention can be expressed in any host cell, e.g., any bacterial cell, any yeast cell, e.g., Pichia pastoris, Saccharomyces cerevisiae or Schizosaccharomyces pombe. Exemplary bacterial cells include any species within the genera Escherichia, Bacillus, Streptomyces, Salmonella, Pseudomonas and Staphylococcus, including, e.g., Escherichia coli, Lactococcus lactis, Bacillus subtilis, Bacillus cereus, Salmonella typhimurium, Pseudomonas fluorescens. Exemplary fungal cells include any species of Aspergillus. Exemplary yeast cells include any species of Pichia, Saccharomyces, Schizosaccharomyces, or Schwanniomyces, including Pichia pastoris, Saccharomyces cerevisiae, or Schizosaccharomyces pombe. Exemplary insect cells include any species of Spodoptera or Drosophila, including Drosophila S2 and Spodoptera Sf9. Exemplary animal cells include CHO, COS or Bowes melanoma or any mouse or human cell line. The selection of an appropriate host is within the abilities of those skilled in the art.

The vector may be introduced into the host cells using any of a variety of techniques, including transformation, transfection, transduction, viral infection, gene guns, or Ti-mediated gene transfer.

Where appropriate, the engineered host cells can be cultured in conventional nutrient media modified as appropriate for activating promoters, selecting transformants or amplifying the genes of the invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof.

If the enzymes of the present invention are isolated and purified the cells can be harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed enzyme can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide.

Cell-free translation systems can also be employed to produce a polypeptide of the invention. Cell-free translation systems can use mRNAs transcribed from a DNA construct comprising a promoter operably linked to a nucleic acid encoding the polypeptide or fragment thereof. In some aspects, the DNA construct may be linearized prior to conducting an in vitro transcription reaction. The transcribed mRNA is then incubated with an appropriate cell-free translation extract, such as a rabbit reticulocyte extract, to produce the desired polypeptide or fragment thereof.

Another aspect of the present invention relates to a method for preparing a compound of formula (I) 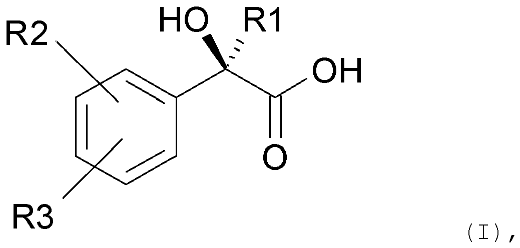 wherein R₁ is a hydrogen, R₂ is selected from the group consisting of -H, -CH₃, -OH, -Cl, -F, -Br, -I, -NO₂, -NH₂, -NRH, -N(R)₂ and - OR, R₃ is selected from the group consisting of -H, -CH₃, -OH, -Cl, -F, -Br, -I, -NO₂, -NH₂, -NRH, -N(R)₂ and -OR, wherein R is an alkyl group comprising 1 to 10 carbon atoms,
the method comprising a step of incubating a compound of formula (II) 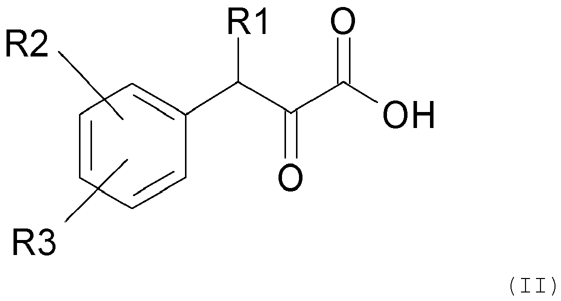 to a variant of a precursor alpha-keto acid dependent enzyme catalyzing the oxidative decarboxylation of an alpha-keto acid and/or to a host cell according to the present invention in an appropriate culture medium.

The compound having formula (I) obtainable with the method of the present invention is in the most preferred embodiment an R-enantiomer.

As mentioned above the compound having formula (I) may be produced by incubating a compound having formula (II) with the variant of the present invention or with a cell overexpressing said variant ("overexpression" in this context means that a recombinant host cell carrying the nucleic acid sequence of the present invention is capable to produce the variant of the present invention to a higher extent than a wild-type host cell). In order to enhance the enantiomeric purity of the product obtained by the present invention the (recombinant) host cell used in the present invention is also able to overexpress S-hydroxmandelate oxidase (HMO). HMO is known to catalyze S-hydroxymandelate to 4-hydroxybenzaldehyde, CO₂ and H₂O₂. Of course it is also possible to add the enzyme directly to a reaction mixture if no host cells are used when producing a compound having formula (I). Methods for overproducing enzymes or proteins within a host cell are well known in the art.

The position of the substituents R₂ and R₃ on the aromatic ring of formula (I) and (II) may be any one of the three positions of ortho-, meta- or para-. Particularly preferred compounds are those compounds which comprise a substituent in the para position of the aromatic ring. Furthermore also preferred are compounds which comprise a substituent on the aromatic ring which is not hydrogen.

In an alternative embodiment of the present invention a host cell may be used which is able to overproduce phenylpyruvate. Such cells are known in the art (see e.g. Mueller U et al. Metab Eng. 8 (2006) :196-208).

A particularly preferred embodiment of the present invention relates to the production of R mandelic acid and derivatives thereof using 4-phenylpyruvate or derivatives thereof as substrate.

In the art several methods for producing R-mandelate and derivatives thereof are known. Most of these methods include chemical synthesis: racemic resolution of chemically synthesized R,S-mandelic acid (racemic modification) by fractional crystallization as described in JP-A-58-177933; chromatography as described in EP 0 098 707; and conversion of the racemic modification into racemic esters followed by racemic resolution thereof by enzymatic asymmetric hydrolysis as described in K. Mori et al. (Tetrahedron 36(1980):91-96); and chemical asymmetric synthesis with the use of a chiral reagent as described in D.A. Evans et al. (J. Am. Chem. Soc., 107(1985):4346-4348). Biological methods therefore include the asymmetric hydrolysis of the abovementioned esters formed by the process of K. Mori et al. and microbiological asymmetric reduction of benzoylformic acid as described in JP-A-57-198096; hydrolysis of R(-)-mandelonitrile and substituted derivatives thereof which are asymmetrically synthesized with the use of D-oxynitrilase as described in US 4,859,784 and US 5,008,192; and asymmetric hydrolysis of mandelonitrile, mandelamide and substituted derivatives thereof with the use of microorganisms belonging to the genus Alcaligenes, Pseudomonas, Rhodopseudomonas, Corynebacterium, Acinetobacter, Bacillus, Mycobacterium, Rhodococcus or Candida as described in EP 0 348 901.

However, each of the racemic resolution methods described above requires a complicated process and a decrease in the yield occurs in each step. The method of D.A. Evans et al. requires a chiral reagent as a catalyst, which is expensive and which does not allow to produce a product with high optical purity.

The above-mentioned biological methods also have some disadvantages. For instance, in the method of JP-A-57-198096 it is difficult to synthesize the substrate for the asymmetric reduction of benzoylformic acid. It is further difficult to maintain the NADH-regeneration system. The D-oxynitrilase method (US 4,859,784 and US 5,008,192) is merely a basic finding that an optically active substance is obtained. The asymmetric hydrolysis method described in EP 0 348 901 requires a post treatment of another optically active substance remaining after the completion of the hydrolysis. In addition, the EP 0 348 901 contains no particular example of the production of a R(-)-mandelic acid derivative from a substituted derivative of mandelonitrile. Therefore, it is unknown whether an R(-)-mandelic acid derivative of a high optical purity can be efficiently obtained or not.

According to a preferred embodiment of the present invention the substrate (compound having formula (II)) is added to the reaction mixture comprising the variant of the present invention and/or host cells expressing said variant in an amount between 0.1 and 50 mM, preferably between 1 mM and 30 mM, more preferably between 5 and 20 mM, most preferably in an amount of 10 mM.

In a preferred embodiment of the present invention the product obtained with the method of the present invention can be isolated from the supernatant or reaction mixture by methods known in the art. Optionally chiral column chromatography may be used additionally to isolate the product.

The present invention is further illustrated in the following figures and examples, however, without being restricted thereto.
Fig. 1 shows a setup for synthesizing R-mandelate in cells from feed stock using a phenylpyruvate overproducing platform.
Fig. 2 shows a process for the production of R-mandelic acid (Wolfgang Aehle, Enzymes in industry: production and applications, 2007, Wiley-VCH Weinheim).
Fig. 3 shows a sequence alignment of Hydroxymandelate synthase of various sources.

### EXAMPLES:

### Example 1: Biochemical Characterization of 2-Hydroxymandelate Synthase and assessment of the substrate spectrum

### 1.1 Materials and methods

### 1.1.1 Chemicals and Enzymes

Sodium phenylpyruvate (PP) was obtained from Fluka (U.S.A.) and 4-hydroxyphenylpyruvic acid (HPP), HPLC standards, p-nitrophenylpyruvic acid, and all other chemicals were purchased from Sigma Aldrich (U.S.A.). Enzymes for molecular biological experiments were purchased from MBI Fermentas GMBH (Germany) if not stated otherwise. p-hydroxy-phenylpyruvic acid was obtained from Sigma Aldrich (USA). p-Fluor-phenylpyruvic acid, p-methylphenylpyruvic acid and p-methoxy-phenylpyruvic acid were synthesized as described as follows:

The three differently substituted derivatives of phenylpyruvate were synthesized from the corresponding benzaldehydes:

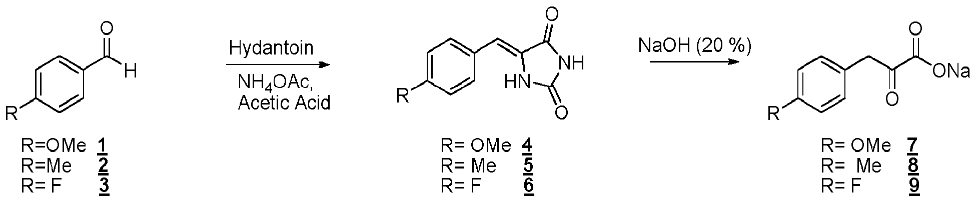

Aldehydes **2** and **3** are commercially available, compound **1** was synthesized from 4-hydroxybenzaldehyde, using iodomethane. From here, the substituted benzaldehydes were converted into their corresponding imidazolidines, using hydantoin and ammonium acetate in acetic acid, affording compounds **2**-**4** in 80-85% yield. The crude products were used directly in the next step, the basic cleavage of the hydantoins. As the products are rather sensitive to moisture and air and NMR showed sufficient purity, the crude alpha-keto acids were lyophilized and stored at -20°C.

Synthetic methods: All non-aqueous reactions were carried out under a positive pressure of argon using oven-dried (100°C) or flame-dried glassware (under vacuum) unless otherwise noted.

Solvents and chemical purification: Dimethyl sulfoxide was dried by distillation from calcium hydride under reduced pressure. Dichloromethane was purified by distillation prior to use. Dry solvents were stored under an argon atmosphere over molecular sieve (4 Ǻ).

All other commercially available reagents were used without further purification. Except if indicated otherwise, reactions were magnetically stirred and monitored by thin layer chromatography using Merck silica gel 60-F254 glass plates. The plates were developed with a mixture of hexane/ethyl acetate or toluene/ethyl acetate. Unless the compound was colored, UV-active spots were detected at longwave UV (254 nm) or shortwave (180 nm). Most plates were additionally treated with one of the following visualization reagents: CAM [H₂SO₄ (conc., 22 mL), phosphormolybdic acid (20 g), Ce(SO₄)₂ (0.5 g), 378 mL H₂O)] or silica gel impregnated with iodine .

**Chromatography:** Preparative column chromatography and flash chromatography was performed with silica gel 60 from Merck (0.040-0.063 µm, 240-400 mesh).

Concentration under reduced pressure was performed by rotary evaporation at 30°C at the appropriate pressure, unless otherwise stated. Yields refer to chromatographically purified and spectroscopically pure compounds, unless otherwise stated.

**NMR spectra:** NMR spectra were recorded either on a Bruker Avance AV 400, DRX 400, or DRX 600 MHz spectrometer. Unless otherwise stated, all NMR spectra were measured in CDCl₃ solutions and referenced to the residual CDCl₃ signal (¹H, δ = 7.26, ¹³C, δ = 77.16). All ¹H and ¹³C shifts are given in ppm (s = singlet, d = doublet, dd = doublet of doublets, t = triplet, q = quartet, m = multiplet, br = broadened signal). Coupling constants J are given in Hz.

### 4-Methoxybenzaldehyde 1

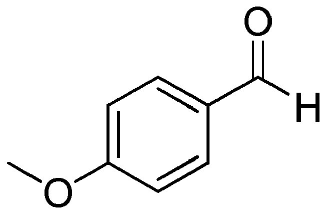

A suspension of 4.5 g (4 eq., 81.8 mmol) KOH in 104 mL of DMSO was stirred for 20 min before 4-hydroxybenzaldehyde (2.5 g, 20.45 mmol) and iodomethane (2 eq, 2.55 ml) were added. Stirring at room temperature was continued until TLC (PE:EE 2:1) showed no remaining starting material. The orange solution was diluted with dichloromethane (300 mL) and poured into ice-water. The aqueous phase was extracted with dichloromethane and the combined organic extracts were washed with water, dried over Na₂SO₄ and evaporated to yield 2.7 g of crude methoxybenzaldehyde (quant.), which was used without further purification.

### Experimental data:

**¹H-NMR** (400MHz, CDCl₃), δ [ppm] 9.88 (s, 1H); 7.83 (d, 2H, 8.86 Hz); 6.99 (d, 2H, 8.86 Hz); 3.88 (s, 3H).

### 5-(4-Methoxybenzylidene)imidazolidine-2,4-dione 4

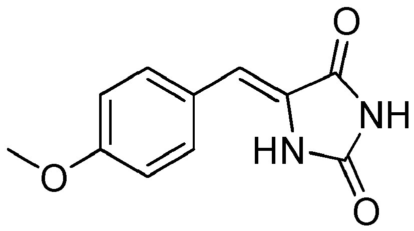

A mixture of ammonium acetate (1.6 g; 1.0 eq), hydantoin (2.0 g; 1.0 eq) and 2.7 g **1** (20.4 mmol) was heated in 18 mL acetic acid under reflux for 5 hours (120 °C). The hot solution was poured into 20 mL of cold water and warmed to room temperature. The yellow precipitate was filtered and washed with small portions of cold water and hexanes. Subsequent drying afforded 3.5 g (16.3 mmol, 80%) of hydantoin **4**.

### Experimental data:

**¹H-NMR** (400MHz, DMSO), δ [ppm] 10.75 (bs, 2H); 7.58 (d, 2H, 8.83 Hz); 6.95 (d, 2H, 8.83Hz); 6.38 (s, 1H); 3.79 (s, 3H).

**¹³C-NMR** (400MHz, DMSO), δ [ppm] 165.63; 159.44; 155.66; 126.11; 125.45 (**¹³C**_{q}); 131.08, 114.30 (2x **¹³C**H) ; 108.64 (**¹³C**H) ; 55.27 (**¹³C**H₃).

### 5-(4-Methylbenzylidene)imidazolidine-2,4-dione 5

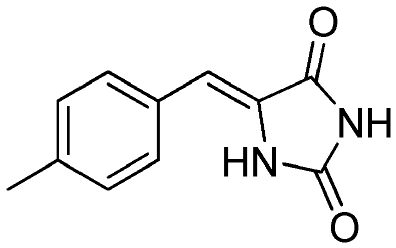

According to the same procedure as the preparation of 5-(4-methoxybenzylidene)imidazolidine-2,4-dione **4**, 1.0 g (8.3 mmol) of tolualdehyde **2**, 840 mg of hydantoin and 2.5 g of ammonium acetate afforded 1.4 g (7 mmol, 84%) of corresponding hydantoin **5**.

### Experimental data:

**¹H-NMR** (400MHz, DMSO), δ [ppm] 10.83 (bs, 2H); 7.52 (d, 2H, 8.25 Hz); 7.22 (d, 2H, 8.25 Hz); 6.39 (s, 1H); 2.33 (s, 3H).

**¹³C-NMR** (400MHz, DMSO), δ□[ppm] 165.59, 155.65, 138.14, 130.12, 127.21 (**¹³C**_{q}); 129.39, 129.36 (2x **¹³C**H) ; 108.49 (**¹³C**H); 20.92 (**¹³C**H₃).

### 5-(4-Fluorobenzylidene)imidazolidine-2,4-dione 6

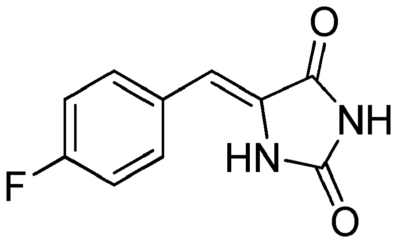

According to the same procedure as the preparation of 5-(4-methoxybenzylidene)imidazolidine-2,4-dione **4**, 2.0 g (16.1 mmol) of 4-fluorobenzaldehyde **3**, 1.6 g hydantoin and 5.0 g of ammonium acetate yielded 2.6 g of compound **6** (12.6 mmol, 79%).

### Experimental data:

**¹H-NMR** (400MHz, DMSO), δ□[ppm] 7.63-7.71 (m, 2H); 7.19-7.28 (m, 2H); 6.41 (s, 1H).

**¹³C-NMR** (400MHz, DMSO), δ [ppm] 162.99, 160.53 (**¹³C**-F) ; 165.71, 155. 95, 129.66, 127. 98 (**¹³C**_{q}) ; 131.56, 131.48, 115.81, 115.60, 107.01 (**¹³C**H).

**¹⁹F-NMR** (600MHz, DMSO), δ [ppm] -112.74.

### Sodium 3-(4-methoxyphenyl)-2-oxopropanoate 7

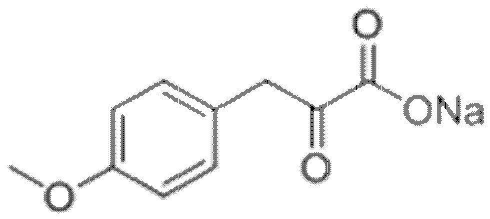

A three necked flask equipped with reflux condenser and septum was charged with 1.5 g of hydantoin **4** and set under argon atmosphere. After adding NaOH (20%, 32 mL), the apparatus was again flushed with argon. The mixture was heated under reflux for 3 hours, then cooled to 0°C and adjusted to pH 7 by slow addition of HCl_{conc}. After addition of 650 mg NaHCO₃, the mixture was extracted with diethyl ether in a liquid-liquid extraction apparatus for 3 hours. Subsequent addition of 9 mL HCl_{conc} and further extraction extracted the product into the organic phase. The solvents were removed *in vacuo*, yielding a yellow oil which was taken up in 70 mL H₂O and readjusted to pH 7 by addition of 1 M NaOH. Freezing with liquid nitrogen and lyophilization gave white crystals of **7** (864 mg, 4 mmol, 61%).

### Experimental data:

**¹H-NMR** (400MHz, D₂O), δ□[ppm] 7.17 (d, 2H, 8.48 Hz); 6.97 (d, 2H, 8.48 Hz); 4.00 (s, 2H); 3.80 (s, 3H).

**¹³C-NMR** (400MHz, D₂O), δ□[ppm] 181.29, 157.72, 157.24, 130.47 (**¹³C**_{q}); 130.70, 114.49 (2x **¹³C**H) ; 55.77 (**¹³C**H₃) ; 43.83 (**¹³C**H₂).

### Sodium 2-oxo-3-p-tolylpropanoate 6

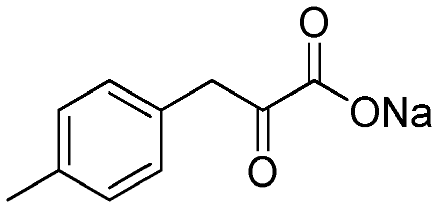

According to the same procedure as preparation of **7**, 1.4 g (6.9 mmol) of hydantoin **5** afforded 916 mg of tolylpyruvate **8** (4.6 mmol, 65%) as a white solid.

### Experimental data:

**¹H-NMR** (400MHz, D₂O), δ□[ppm] 7.19 (d, 2H, 7.19 Hz); 7.12 (d, 2H, 7.19 Hz); 4.00 (s, 2H); 2.27 (s, 3H).

**¹³C-NMR** (400MHz, D₂O), δ [ppm] 203.10, 138.11, 137.50, 130.02 (**¹³C**_{q}); 130.16, 129.82 (2x **¹³C**H); 45.78 (**¹³C**H₂), 20.50 (**¹³C**H₃).

### Sodium 3-(4-fluorophenyl)-2-oxopropanoate 9

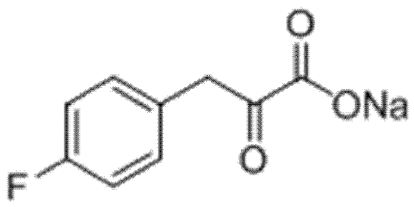

Analogously to the preparation of **7**, 2.0 g (9.7 mmol) of hydantoin **6** yielded 1.22 g (6.0 mmol, 62%) of compound **9** as a white solid.

### Experimental data:

**¹H-NMR** (400MHz, D₂O), δ□[ppm] 7.23-7.33 (m, 2H); 7.13-7.22 (m, 2H); 4.13 (s, 2H).

**¹³C-NMR** (400MHz, D₂O), δ□[ppm] 204.32, 181.62, 162.92, 130.13 (**¹³C**_{q}); 131.94, 131.86, 115.96, 115.75 (**¹³C**H) ; 45.40 (**¹³C**H₂).

**¹⁹F-NMR** (600MHz, D₂O), δ [ppm] -116.80.

### 1.1.2 Strain and media

Streptomyces coelicolor A3 (2) was obtained from 'Deutsche Sammlung von Mikroorganismen und Zellkulturen GmbH' (DSMZ- ID no. 40783). The strain was kept on the Streptomyces 'medium 65' plates, according to the maufacturer's recommendation. E. coli BL21(DE3) from Stratagene (U.S.A.) and E. coli Rosetta 2 from Merck KGa (Germany) were used as an expression host for 4-hydroxymandelate synthase (Hms). E. coli cells were kept on agar plates of Luria broth medium supplemented with 50 mg/mL kanamycin and with 0.05 mg/L of ampicillin and 0.06 mg/L of cloramphenicol for for E.coli BL21 (DE3) and Rosetta 2 cells respectively.

### 1.1.3 Cloning of 4-hydroxymandelate synthase Hms from Streptomyces coelicolor A3 (2)

Genomic DNA from S. coelicolor A3 (2) was obtained using the 'DNA purification Wizard' of Promega (U.S.A) according to the manufacturer's procedure from cells that had been grown in 'medium 65' liquid broth for 24 hours at 30°C, harvested and washed with ice-cold 50 mM KH₂PO₄/K₂HPO₄ buffer, pH 7.5. Cells were broken in the presence of 20 mg/mL lysozyme.

Primers to amplify Hms were designed based on the annotated sequence information of S. coelicolor A3 (2) genomic DNA. a) C-terminally tagged Hms (Hms-C): the forward primer 5' CTA TAC ATA TGC TCC CTC CTT TCC CCT TCC TTC 3' (SEQ ID No. 11) was engineered with an NdeI site (underlined) which overlapped the initiation codon of hms. The reverse primer 5' ATA GAG GAT CCT TA**T TTT TCG AAC TGC GGG TGG CTC CAA GCG CT**T CGG CCG GCC ACT TCC CG 3' (SEQ ID No. 12) downstream of the stop codon, was designed with a BamHI site (underlined) and a streptavidine affinity tag (in bold).

Amplification was performed in a total volume of 50 µL with 5 Units/µl of Taq polymerase (Fermentas GMBH Germany), 50 ng of chromosomal DNA from S. coelicolor as a template, 50 pmol of each primer, 1 µL of dNTPs (1 mM each), 5 µL of Taq polymerase buffer, 4% DMSO, applying 30 cycles at 94°C (30 s), 68°C (30 s) and 72 °C (60 s). b). The amplification was performed in a final volume of 50 µl with 2.5 Units/µl of Pfu polymerase (Fermentas GMBH Germany), using 100 ng of the hms bearing vector pKYB1 (vide supra) resulting from above, 25 pmol of each primer 1 µL of dNTPs (200 µM each) 5 µL of Pfu polymerase buffer, 4% DMSO and applying 30 cycles at 95 ° C (90 s), 65 ° C (30 s) and 72 ° C (90 s) .

DNA fragments from a) were analysed and purified using agarose gel electrophoresis, extracted using QIAquick PCR purification kit protocol (Quiagen, Germany) and cloned into the NdeI/BamHI restriction sites of the expression vector pKYB1 (Stratagene, USA), thus allowing inducible expression of recombinant Hms from the strong T7 promoter. The DNA fragments from b) were cut with BpiI (Fermentas GMBH Germany) and ligated into BsaI (Fermentas GMBH Germany) cut vector pASK-IBA 7 plus (IBA, USA) using standard procedures.

*1.1.4 Protein production, purification and reactivation* C-terminally tagged Hms: E. coli BL21(DE3) cells bearing the Hms expression construct were grown in a culture medium allowing the production of an Fe(II)-oxygenase (Hofer, H et al. J Biotechnol 107(2004):73-81). N-terminally tagged Hms: E. coli Rosetta 2 cells bearing the Hms expression construct were grown in a culture medium with 0.05 mg/L of ampicillin and 0.06 mg/L of cloramphenicol as a selection marker. When the main culture showed an OD₆₀₀ of 0.8 expression was induced by adding 200 µg/L of anhydrotetracycline. Cells were grown for 24 hours at 18°C and then harvested by centrifugation at 6000 rpm at 4°C for 20 min.

Cell pellets were resuspended in ∼30 ml of Tris/HCl buffer (20mM, pH 7.5) and the ice-cold suspension was subjected to 2 cycles of the cells disruption in the French Press at 8500 kPa and subsequent centrifugation 70,000 g for 2 hours.

Affinity purification was carried out at 4°C using a 5 ml Strep-Tactin column (IBA, Germany) according to the manufacturer' s protocol, as described previously (Straganz, G. D.; et al. Biocatalysis and biotransformation 23(2005):261-269). Purified protein was then concentrated 20-fold, using Vivaspin centrifugation concentration tubes (Sartorius Stedim Biotech S.A., France) and subjected to three cycles of buffer exchange via size exclusion chromatography using a Nap-25^{™} column (GE Healthcare, U.K.). Protein purity was assessed by SDS PAGE.

### 1.1.5 Protein concentration and iron content

Protein concentrations were determined via UV/Vis spectroscopy by measuring the absorbance of an appropriately diluted protein solution (K₂HPO₄/KH₂PO₄, 50 mM, pH 7.5) at 280 nm and calculating the concentration with a theoretical ε, which had been determined based on the sequence information. To test the presence of iron in the enzyme solution, the adapted FereneS reaction was used (Straganz, GD et al. J. Am. Chem. Soc. 127(2005):12306-14). All measurements were performed in Tris/HCl buffer, 20 mM, pH 7.5. Fe(II) concentrations in the protein preparations were determined spectrophotometrically by monitoring the formation of the colored Fe(II)-FereneS chelate complex, as described previously The spectro-photometric method was validated using ICP-MS.

### 1.1.6. Enzyme activity assays

Dioxygen consumption was continuously monitored using a micro-optode O₂ sensor (Microtox TX3-AOT, Presens GmbH,Regensburg, Germany). Assays were generally performed at 25°C in air saturated 20 mM Tris/HCl buffer, pH 7.5 with enzyme concentrations of approx. 20 µM. To start the reaction, substrate aliquots were added from 5 mM stock solutions of the respective substrate to the desired end-concentration in a total volume of 500 µL. Initial rates were determined by linear regression analysis using the initial, linear part of the O₂ depletion curve. O₂ consumption rates from blanks that contained substrate in the absence of enzyme were subtracted as necessary. Specific activities were obtained by relating measured rates of O₂ depletion to the protein derived Fe(II) content in the assay.

### 1.1.7 Chromatographic methods

Product analysis via HPLC: Cleavage products of PP by HMS were determined on an HPLC system (Merck-Hitachi La Chrom, Germany) equipped with a UV-detector, using the chiral column Chiralpak AD-RH (Diacel, France). 88 % Water + 0.1 % trifluoroacetic acid (TFA) and 12 % acetonitril (ACN) + 0.1% TFA were used as eluents. Analyses were performed at 30°C column temperature using a flow rate of 0.5 mL/min. The elution of substrate and products was monitored at 210 nm.

The identity of reaction products from enzymatic conversions was confirmed via HPLC-MS with a variable wavelength detector, which operated at an analytical wavelength of 210 nm, and an MS detector, which was used in negative mode and with APCI ionization.

Gel Filtration: The size of Hms was estimated via gel filtration using ∼ 1 mg of purified protein with a Superdex 200 gel filtration column (25ml; Amersham Biosciences) under standard conditions, using 20 mM MES buffer, pH 7.5, and 0.15 M NaCl as eluent. A flow rate of 0.4 mL/min was applied. Calibration was performed using a Gel Filtration Standard Kit (Bio-Rad, U.S.A.).

### 1.1.8 Circular dichroism spectroscopic studies

The far-UV CD spectra were recorded with a spectropolarimeter at 25°C using 0.02 and 0.10 cm path-length cylindrical cells. The instrument parameters were operated with a step resolution of 0.2 nm, scan speed of 50 nm/min, response was 1 s and bandwidth was 1 nm. Enzymes were prepared in Tris/HCl buffer pH 7.5 20 mM at a protein concentration of 3.0 mg/mL. Spectra obtained in the wavelength range 300-190 nm were averaged and corrected by a blank spectrum lacking the enzyme before converting the signal into mean residue ellipticity.

### 1.2. Results

### 1.2.1 Isolation of HMS from Streptomyces coelicolor S2 (A3)

The ∼1200 bp product was amplified from genomic DNA from Streptomyces coelicolor A3 (2) was cloned into an expression vector, overexpressed with a C-terminal tag, and purified as outlined in Material and Methods. The resulting protein showed >95% purity, as estimated from the SDS gel. The size of the protein monomer estimated from SDS gel was ∼ 41 kDa, which corresponds to the theoretical mass of Hms of 41,412 Da.

### 1.2.2 Biochemical characterization

Hms was characterized regarding the content of ferrous iron, which is crucial for its catalytic functionality. Iron contents in the enzyme preparations were determined spectroscopically by monitoring iron detachment at 25°C and typically gave values in the range of ∼40% (vide supra) related to the metal binding site.

Low iron contents are due to iron lability, which is typically observed in mononuclear nonheme Fe(II) enzymes. Iron detachment rates were determined at 21°C and gave values of 0.0006 s⁻¹ . This is comparable with the rate reported in literature for Hppd, of 0.0006 s-1 at 4 °C (Johnson-Winters, K. et al.Biochemistry 42 (2003) :2072-80) .

Preparations of purified C-terminally tagged Hms showed single bands with an apparent molecular mass of ∼ 40 kDa with SDS/PAGE under both, reducing and non-reducing conditions. Gel filtration using a Superdex size-exclusion column showed a single homogenous peak in the elution profile that corresponded to a molecular mass of ∼ 45 kDa, which suggests that HMS is present in its monomeric form under the conditions applied. The secondary structure composition of Hms was assessed via CD spectroscopy. C-terminally tagged HMS showed a composition of 16% alpha helix, 33% beta sheet and 20% of random coil. The enzymatic activity of Hms towards its native substrate was determined under standard conditions (Tris/HCl buffer pH 7.5 20 mM) under air saturating conditions. A specific activity of 4.4 s⁻¹ was determined for conversion HPP by HMS. Conditions of the activity assay were varied, in order to assess the impact of variable parameters, which have previously been applied in enzymatic activity assays of Hppd and Hms and may have a potential effect on activity. However, neither a pH shift to 7.0 nor a change of the buffer system (HEPES 20 mM, pH 7.0) or the addition of 1 mM DTT had significant effects on the enzymatic activity of Hms, which remained the same throughout the conditions tested, within the experimental error of 10 %.

### 1.2.3 Substrate spectrum of HMS

In order to assess the substrate spectrum, a screening for enzymatic activity of Hms towards a range of commercially available α-oxo acids was performed. Therefore, enzymatic dioxygen consumption rates were measured in the presence of the respective substrate (5 mM). Aliphatic substrates generally showed activities that were 1000-fold lower than for the native substrate as detailed in Table 1.

**Table 1: Specific activities of Hms towards a range of 2-oxo-compounds. Reactions were performed in Tris buffer (20 mM, pH 7.5), at 25°C at air saturation. Substrate concentration was 5 mM, Hms was used at a concentration of 100 µM. Standard deviation was < 15 % in all cases. The limit of detection under assay conditions was 10⁻⁵ s⁻¹.**

| substrate | catalytic activity(s⁻¹) |
|---|---|
| 4-hydroxyphenylpyruvic acid | 4.5 |
| 2-oxo-4-methylthiobutyric acid | 0.007 |
| 3-methyl-2-oxo-butyric acid | 0.0006 |
| 2-oxo-butyric acid | < 10⁻⁵ |
| 2-oxo-octanoic acid | 0.001 |
| 2-oxoglutaric acid | < 10⁻⁵ |
| 4-methyl-2-oxo-pentanoic acid | < 10⁻⁵ |
| 2-oxo-valeric acid | < 10⁻⁵ |

The reactivity of HMS towards a range of aromatic oxo-acids was assessed, whereby the impact of substituents in the para-position of the aromatic ring regarding electronic structure and size was probed. Therefore, p-fluor, p-methyl- and p-methoxy-phenylpyruvate were synthesized, while the p-nitro-analogues and phenylpyruvate were commercially available. Where activities were high enough to allow a steady state kinetic analysis, specific activities (kcat) and Michaelis Menten constants (Km) were determined (the assays were performed at air saturation (=260µM dioxygen concentration, therefore all kinetic constants are apparent)). Reactivities towards aromatic substrates were generally higher than for their aliphatic counterparts and showed specific activities that were 5-fold - in the case of p-methoxy-PP, which bears an electron pushing substitutent- to ∼100 fold reduced towards p-fluor-PP and p-nitro-PP, which display electron withdrawing groups. The substrate analogue 2-oxo-4-phenylbutanoic acid (OPB) was accepted as a substrate by HMS with a specificity that was comparable to phenylpyruvate-type substrates. Results are summarized in Table 2.

**Table 2: Specific activities of Hms towards a range of aromatic 2-oxo-compounds. Reactions were performed in Tris buffer (20 mM, pH 7.5), at 25°C at air saturation. Steady state kinetic constants and the enantiomeric excess of the respective conversion are given.**

| substrate | *K*m | *k*_{cat} | e.e. |
|---|---|---|---|
| | (µM) | (s⁻¹) | % |
| 4-Hydroxyphenylpyruvic-acid | 220±80 | 4.5±0.5 | >90 |
| 4-Phenylpyruvic acid | 353+87 | 0.88±0.09 | 99.2 |
| 4-Methylphenylpyruvic acid | n.d. | 0.13±0.02 | >95 |
| 4-Methoxyphenylpiruvic acid | 160±70 | 0.96±0.12 | 0.1 |
| 4-Fluor-phenylpyruvic acid | n.d. | 0.077±0.004 | 76 |
| 2-Oxo-4-phenylbutanoic acid | 35+20 | 0.17±0.02 | > 90 |
| p-Nitrophenylpyruvic acid | n.d. | 0.035±0.007 | n.d. |

In order to study the impact of the substrate structure on the enantiospecificity of the reaction, enantiomeric ratios of the conversion products were assessed using HPLC and GC-MS, respectively. The conversion of the native substrate HPP yielded >95% of the S enantiomer. Equally high enantiospecificities were obtained for the conversion products of PP (%), p-methyl-PP and, also for 4-phenyl-2-oxo-butanoate.

A quantitation of reaction products showed that the recovery of the reaction products as mandelate was > 90 % in the conversions of, PP, p-methyl-PP, p-methoxy-PP, p-fluor-PP and OPB, as confirmed via HPLC-MS.

### 1.3 Summary

HMS from Streptomyces coelicolor S2(A3) was biochemically characterized and its substrate spectrum was assessed. HMS accepted substrates that display an aromatic substituent with comparably high reactivities, whereby it was not limited to PP-type structures but also accepted the longer substrate OPB. In-silico docking analyses revealed a binding pocket which was generally occupied by the aromatic substituent in all docking analyses. By contrast, aliphatic residues were converted at very low rates or not at all.

Notably, more hydrophobic aliphatic substrates, such as 2-oxo-octanoic acid and 2-oxo-4-methylthiobutyric acid induce some O₂ consumption, while hydrophilic compounds have no detectable effect.

### Example 2: 4-Hydroxymandelate synthase (HMS) variants

S-4-Hydroxymandelate synthase (HMS) is a dioxygenase that oxidizes 4-hydroxy-phenylpyruvate (PP) to S-4-hydroxy-mandelate in the course of Calcium Dependent Antibiotic (CDA) biosynthesis and related pathways, but HMS also accepts phenylpyruvate (PP) and converts it to S-mandelate with high enantiomeric purity (≥99%). By mutating specific amino acid residues within the enzyme this enantispecificity could be inverted so that phenylpyruvate and derivatives will be converted to the R-analogues with an enantiospecificity of up to >97%.

In the present example an HMS from *Streptomyces coelicolor* 2(A3) was used. In example 1, where the enzyme was purified and its substrate structure was characterized, it could be shown that a remarkable dependence of enantio-specificity on the substrate structure (Table 2). On the basis of the structure of HMS from *Arabidopsis orientalis* a model was built which allowed the introduction of single point variations in silico within the aforementioned HMS from *Streptomyces coelicolor* 2(A3). These mutations resulted in the destruction of the (S)-4-hydroxymandelate binding pocket and/or in the creation of space for (R)-4-hydroxy-mandelate ligation. Variants were constructed and characterized regarding their enantiospecificity. Some variants were combined to give doulbe- and triple mutants. Due to this strategy a HMS variant could be designed that converts PP to R-mandelate with 97.4 % enantio-purity with an activity that is ∼80% of wild type enzyme, other substrates are converted to products with even higher enantiopurity. Kinetic data and enantispecificities of the respective variants are outlined in Table 3.

**Table 3:**

| **Variant** | **Km** | **kcat** | **% R** | **recovery** | **Substrate** | **Product** |
|---|---|---|---|---|---|---|
| | **(mM)** | **(s⁻¹)** | | | | |
| Wild type | 0.35 | 0.9 | 0.4 | > 90 % | phenylpyruvate | mandelate |
| V223F | 0.027 | 1.1 | 65.1 | > 95 % | phenylpyruvate | mandelate |
| V223W | 0.23 | 0.5 | 2.2 | > 80 % | phenylpyruvate | mandelate |
| V223M | 0.23 | 1.9 | 14.6 | > 95 % | phenylpyruvate | mandelate |
| S221M | 0.33 | 1.9 | 88.5 | > 95 % | phenylpyruvate | mandelate |
| I236F | 2.0 | 0.9 | 0.6 | > 60 % | phenylpyruvate | mandelate |
| Y359A | 0.27 | 4.8 | 15.8 | > 95 % | phenylpyruvate | mandelate |
| V223F Y359A | 1.0 | 2.1 | 93.1 | > 95 % | phenylpyruvate | mandelate |
| S221M_Y359A | 0.24 | 1.1 | 94.6 | > 95 % | phenylpyruvate | mandelate |
| S221M_V223M_ Y359A | 0.11 | 0.6 | 97.4 | > 95 % | phenylpyruvate | mandelate |
| S221M_V223F_ Y359A | 0.12 | 1.6 | 95.0 | > 95 % | phenylpyruvate | mandelate |
| Wild type | 0.16 | 1.0 | 50 | > 99 % | p-Methoxy-phenylpyruvate | p-methoxy-mandelate |
| V223F | n.d. | n.d. | > 99 | > 95 % | p-Methoxy-phenylpyruvate | p-methoxy-mandelate |

Kinetic analysis was performed via measuring O₂ depletion. Kinetic parameters are apparent, as measurements were performed at air saturation. Product analysis was performed via HPLC (method is outlined in Appendix B) and confirmed via GC-MS. It could be shown that the enzyme converts phenylpyruvate analogues as a whole cell system, when a substrate analogue (e.g. p-F-phenylpyruvate) was added to cultures of E.coli BL21 cells overexpressing HMS variant S221M_V223M_Y359A. The overexpression was induced with IPTG as described in Material and Methods. To the cell culture simultaneously solid p-Fluor-phenylpyruvate was added at an end concentration of 10 mM. After 24 hours, cells were harvested via centrifugation and the supernatant was subjected to chiral HPLC-MS. 1mM p-Fluor-R-mandelate and no respective S-enantiomer and no residual substrate was found in the supernatant.

It turned surprisingly out that the mutation of Ile236 of HMS *Streptomyces coelicolor* 2(A3) did not influence the activity of the enzyme in relation to its capability to produce R- and S-enantiomers from a substrate (see Table 3). This result was unexpected since also Ile236 is part of the binding pocket of a wild-type HMS in which the S enantiomer of an alpha-keto acid can be bound.

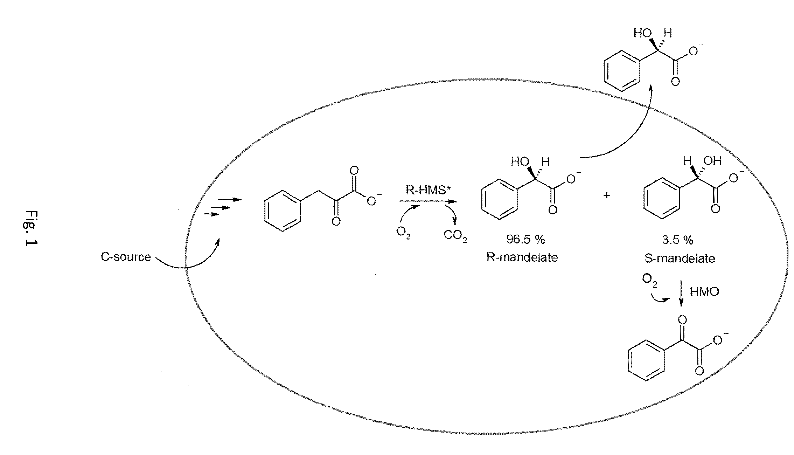

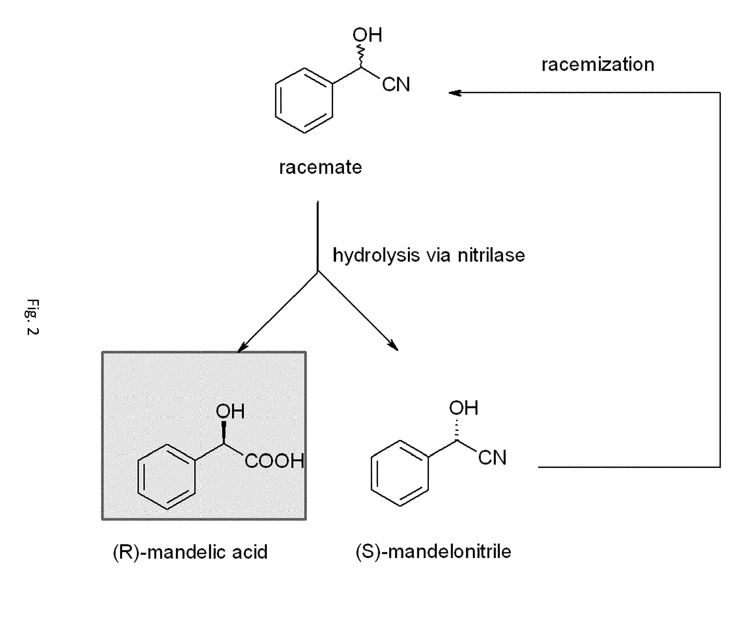

The enzyme can be used for the biosynthesis of R-Mandelate from feed stock e.g. using a phenylpyruvate overproducing platform as outlined in Fig. 1 and in principle analogy to work from Wubboltz and co-workers (Müller, U et al. Metab. Eng. 8(2006):196-208), who used an engineered E. coli platform that overproduces phenylpyruvate for-D phenylglycine biosynthesis using wildtype S-HMS and S-p-hydroxymandelate oxidase and transaminase. In analogy, the operational enantio-specificity of the herein described engineered R-HMS could be further enhanced by co-expression of 4-hydroxy-mandelate synthase, which will oxidize residual S-HMS (Fig. 1). This process is more straightforward than currently used industrial R-mandelate processes - e.g. the BASF process implemented in 2000 (Fig. 2), which includes multiple steps and requires chemical synthesis of the substrate using toxic cyanide.

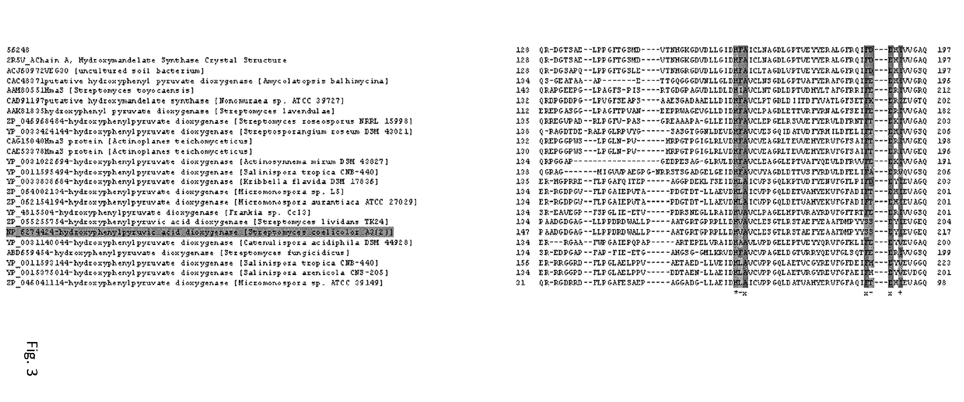

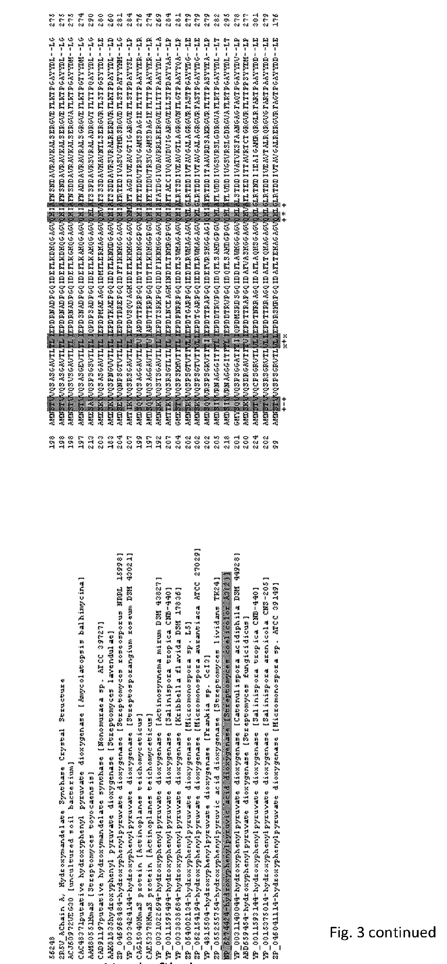

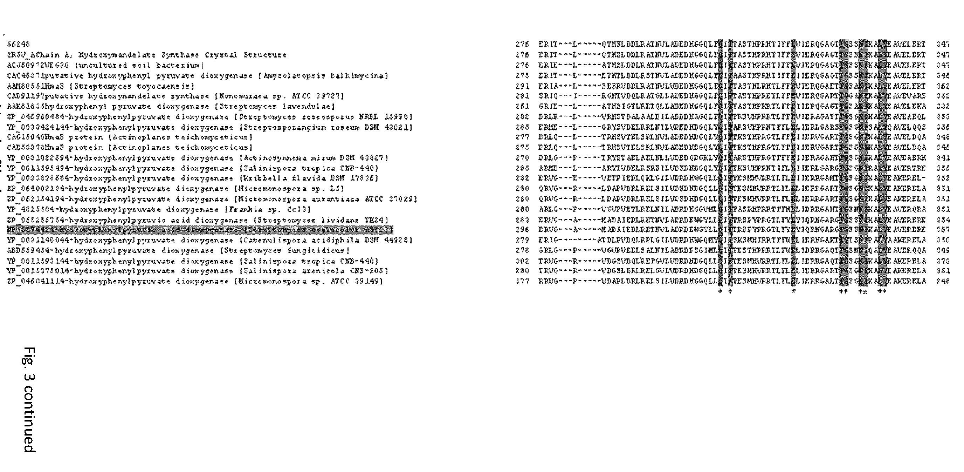

The active site of HMS is very strongly conserved (Fig. 3) and analogous substitutions in the active site of other HMS will yield the same effect.

### 2.1 Materials and methods

### 2.1.1 Site directed mutagenesis

Mutations of HMS (wild-type *Streptomyces coelicolor* 2(A3) sequence; SEQ ID No. 1) were carried out according to the two-stage PCR method reported by Wang et al. (Biotechniques. 26(1999):680-682). using the plasmid vector pKYB1/HmSSc-C as the template. Mutations were introduced at positions 221, 223, 236 and/or 359 (Ser221Met, Va1223Phe, Va1223Trp, Va1223Met, Ile236Ala, Ile236Phe, Tyr359Ala) (see Table 3) using *Streptomyces coelicolor* A3 (2) numbering (SEQ ID No. 1). The two-stage PCR technique is based on two separate primer extension reactions, one for each forward and reverse primer (stage 1).

After the first run 25 µL of each related primer extension reaction-mixture were pooled, and the second temperature cycle (with 18 cycles of the main step) was started. To assure, that parental DNA is removed, 25 µL of the PCR products were treated with 1 µL DpnI (37°C, 1 hour), which digests methylated and hemimethylated DNA. Subsequently the samples were desalted and concentrated by QIAquick PCR purification kit (Quiagen GmbH, Germany) .

Sequence of the primers for introducing mutations Ser221Met, Va1223Phe, Va1223Trp, Va1223Met, Ile236Ala, Ile236Phe and/or Tyr359Ala into HMS of *Streptomyces coelicolor* 2(A3):
Primer Sequence 5'→ 3'

- HMS_Val_223_Trp_fw: GATGGACTCCATCTGGGTGCGCAACG (SEQ ID No. 13)
- HMS_Val_223_Trp_rev: CGTTGCGCACCCAGATGGAGTCCATC (SEQ ID No. 14)
- HMS_Val_223_Met_fw: GATGGACTCCATCATGGTGCGCAACG (SEQ ID No. 15)
- HMS_Val_223_Met_rev: CGTTGCGCACCATGATGGAGTCCATC (SEQ ID No. 16)
- HMS_Val_223_Phe_fw: GATGGACTCCATCTTTGTGCGCAACG (SEQ ID No. 17)
- HMS_Val_223_Phe_rev: CGTTGCGCACAAAGATGGAGTCCATC (SEQ ID No. 18)
- HMS_Phe359Ala_FW: CAACATCAAGGCCCTGGCCGAGGCCGTCGAGCG (SEQ ID No. 19)
- HMS_Phe359Ala_REV: CGCTCGACGGCCTCGGCCAGGGCCTTGATGTTG (SEQ ID No. 20)
- HMS_Ser_221_Met_fw: GATGGACATGATCGTCGTGCGCAACG (SEQ ID No. 21)
- HMS_Ser_221_Met_rev: TGCGCACGACGATCATGTCCATCGCCTGC (SEQ ID No. 22)
- HMS_Ile_236_Phe_fw: CCTTCACGCTCTTTGAACCGGACG (SEQ ID No. 23)
- HMS_Ile_236_Phe_rev: CGTCCGGTTCAAAGAGCGTGAAGG (SEQ ID No. 24)

For Double mutations at positions 223 and 221:
- Ser221Met_ Val223Phe_fw: GATGGACATGATCTTTGTGCGCAACG (SEQ ID No. 25)
- Ser221Met_Val223Phe_rev: TGCGCACAAAGATCATGTCCATCGCCTGC (SEQ ID No. 26)
- Ser221Met_Val223Met_fw: GATGGACATGATCATGGTGCGCAACG (SEQ ID No. 27)
- Ser221Met_Val223Met_fw: TGCGCACCATGATCATGTCCATCGCCTGC (SEQ ID No. 28)

## Claims

**1.** A variant of an alpha-keto acid dependent enzyme catalyzing the oxidative decarboxylation of an alpha-keto acid having an amino acid sequence varying from that of a precursor alpha-keto acid dependent enzyme catalyzing the oxidative decarboxylation of an alpha-keto acid, said precursor enzyme comprising
- a motif (A) X₁X₂X₃X₄(X)ₙFGX₅X₆NX₇X₈X₉X₁₀X₁₁, wherein X₁ is serine, X₂ is an amino acid residue selected from the group consisting of threonine, lysine, isoleucine, arginine and glutamine, X₃ is valine or alanine and X₄ is an amino acid residue selected from the group consisting of valine, methionine and isoleucine, X₅ and X₆ are independently any amino acid residue, X₇ is isoleucine or phenylalanine, X₈ is an amino acid residue selected from the group consisting of lysine, glutamine, arginine and threonine, X₉ is an amino acid residue selected from the group consisting of alanine, serine and glycine, X₁₀ is leucine or isoleucine, X₁₁ is tyrosine or phenylalanine, X is any amino acid residue, and n is an integer between 100 and 150, and
- a metal ion binding motif (B) H(X₁₂)ₘH(X₁₃)ₒE, wherein X₁₂ and X₁₃ are independently any amino acid residue, m and o are independently an integer between 70 and 90,
wherein amino acid residue X₁ and/or amino acid residue X₃ within motif (A) of the variant is an amino acid residue having a molecular volume of more than 150 Å³ and/or amino acid residue X₁₁ within motif (A) of the variant has an amino acid residue having a molecular volume of less than 120 Å³.

**2.** Variant according to claim 1, **characterised in that** amino acid residue X₁ and/or amino acid residue X₃ is selected from the group consisting of arginine, histidine, isoleucine, leucine, lysine, methionine, phenylalanine, tryptophan and tyrosine.

**3.** Variant according to claim 1 or 2, **characterised in that** amino acid residue X₁ and/or amino acid residue X₃ is selected from the group consisting of phenylalanine, tryptophan and methionine, preferably phenylalanine or methionine.

**4.** Variant according to any one of claims 1 to 3, **characterised in that** the amino acid residue X₁₁ is selected from the group consisting of alanine, asparagine, aspartic acid, cysteine, glycine, proline, serine and threonine, preferably serine, glycine or alanine, more preferably alanine.

**5.** Variant according to any one of claims 1 to 4, **characterised in that** n is an integer between 110 and 140, preferably between 115 and 130, more preferably between 120 and 125.

**6.** Variant according to any one of claims 1 to 5, **characterised in that** m and o are independently an integer between 75 and 85, preferably between 78 and 82.

**7.** Variant according to any one of claims 1 to 6, **characterised in that** X₈ is lysine or glutamine and/or X₁₀ is leucine.

**8.** Variant according to any one of claims 1 to 7, **characterised in that** the wild-type alpha-keto acid dependent enzyme catalyzing the oxidative decarboxylation of an alpha-keto acid is a hydroxyphenylpyruvate dioxygenase or a hydroxylmandelate synthase.

**9.** Nucleic acid molecule encoding a variant of a wild-type alpha-keto acid dependent enzyme catalyzing the oxidative decarboxylation of an alpha-keto acid according to any one of claims 1 to 8.

**10.** Vector comprising a nucleic acid molecule according to claim 9.

**11.** Host cell comprising a nucleic acid molecule according to claim 9 or a vector according to claim 10.

**12.** Method for preparing a compound of formula (I) 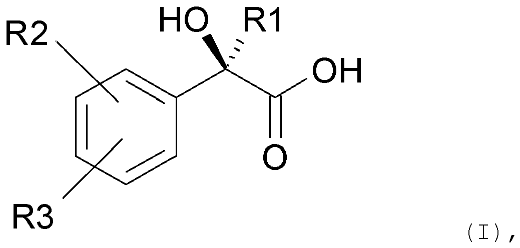 wherein R₁ is a hydrogen, R₂ is selected from the group consisting of -H, -CH₃, -OH, -Cl, -F, -Br, -I, -NO₂, -NH₂, -NRH, -N(R)₂ and - OR, R₃ is selected from the group consisting of -H, -CH₃, -OH, -Cl, -F, -Br, -I, -NO₂, -NH₂, -NRH, -N(R)₂ and -OR, wherein R is an alkyl group comprising 1 to 10 carbon atoms,
the method comprising a step of incubating a compound of formula (II) 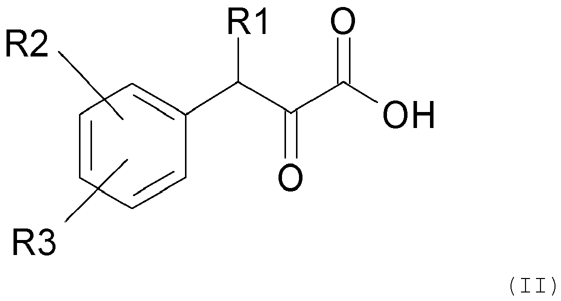 to a variant of a precursor alpha-keto acid dependent enzyme catalyzing the oxidative decarboxylation of an alpha-keto acid according to any one of claims 1 to 8 and/or to a host cell according to claim 11 in an appropriate culture medium.

**14.** Method according to claim 13, **characterised in that** the host cell overexpresses S-hydroxmandelate oxidase (HMO).


SEQUENCE LISTING

<110>  TECHNISCHE UNIVERSITÄT GRAZ

<120>  Enzyme variants

<130>  R 60112

<160>  30

<170>  PatentIn version 3.5

<210>  1
<211>  371
<212>  PRT
<213>  Streptomyces coelicolor A3(2)

<400>  1

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Ser Ile Val Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Tyr Glu Ala Val Glu Arg Glu Arg Glu Val
        355                 360                 365

Ala Gly Arg
    370

<210>  2
<211>  371
<212>  PRT
<213>  Artificial Sequence

<220>
<223>  V223F variant of HMS of Streptomyces coelicolor A3(2)

<400>  2

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Ser Ile Phe Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser

340                             345                             350

Ser Asn Ile Lys Ala Leu Tyr Glu Ala Val Glu Arg Glu Arg Glu Val
        355                 360                 365

Ala Gly Arg
    370

<210>  3
<211>  371
<212>  PRT
<213>  Artificial Sequence

<220>
<223>  V223W variant of HMS of Streptomyces coelicolor A3(2)

<400>  3

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser

195                 200                 205

Ser Glu Tyr Ile Glu Val Gly Glu Gln Ala Met Asp Ser Ile Trp Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Tyr Glu Ala Val Glu Arg Glu Arg Glu Val
        355                 360                 365

Ala Gly Arg
    370

<210>  4
<211>  371
<212>  PRT
<213>  Artificial Sequence

<220>
<223>  V223M variant of HMS of Streptomyces coelicolor A3(2)

<400>  4

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser

50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Ser Ile Met Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Tyr Glu Ala Val Glu Arg Glu Arg Glu Val
        355                 360                 365

Ala Gly Arg
    370

<210>  5
<211>  371
<212>  PRT
<213>  Artificial Sequence

<220>
<223>  Y359A variant of HMS of Streptomyces coelicolor A3(2)

<400>  5

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Ser Ile Val Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Ala Glu Ala Val Glu Arg Glu Arg Glu Val
        355                 360                 365

Ala Gly Arg
    370

<210>  6
<211>  371
<212>  PRT
<213>  Artificial Sequence

<220>
<223>  S221M variant of HMS of Streptomyces coelicolor A3(2)

<400>  6

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Met Ile Val Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr

325                 330                 335

Leu Phe Tyr Glu Tyr Ile Gln Arg Asn Gly Ala Arg Gly Phe Gly Ser
            340                 345                 350

Ser Asn Ile Lys Ala Leu Tyr Glu Ala Val Glu Arg Glu Arg Glu Val
        355                 360                 365

Ala Gly Arg
    370

<210>  7
<211>  371
<212>  PRT
<213>  Artificial Sequence

<220>
<223>  V223F_Y359A variant of HMS of Streptomyces coelicolor A3(2)

<400>  7

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg

180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Ser Ile Phe Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Ala Glu Ala Val Glu Arg Glu Arg Glu Val
        355                 360                 365

Ala Gly Arg
    370

<210>  8
<211>  371
<212>  PRT
<213>  Artificial Sequence

<220>
<223>  S221M_Y359A variant of HMS of Streptomyces coelicolor A3(2)

<400>  8

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala

        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Met Ile Val Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Ala Glu Ala Val Glu Arg Glu Arg Glu Val
        355                 360                 365

Ala Gly Arg
    370

<210>  9
<211>  371
<212>  PRT
<213>  Artificial Sequence

<220>
<223>  S221M_V223F_Y359A variant of HMS of Streptomyces coelicolor A3(2)

<400>  9

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Met Ile Phe Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp
305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Ala Glu Ala Val Glu Arg Glu Arg Glu Val
        355                 360                 365

Ala Gly Arg
    370

<210>  10
<211>  371
<212>  PRT
<213>  Artificial Sequence

<220>
<223>  Y359A_S221M_V223M variant of HMS of Streptomyces coelicolor A3(2)

<400>  10

Met Leu Pro Pro Phe Pro Phe Leu His Trp Arg Ala Ala Met Pro Pro
1               5                   10                  15

Ser Asp Ile Ala Tyr Ala Glu Leu Tyr Val Ala Asp Asp Arg Glu Ala
            20                  25                  30

Ser Gly Phe Leu Val Asp Ser Leu Gly Phe Val Pro Leu Ala Val Ala
        35                  40                  45

Gly Pro Ala Thr Gly Thr His Asp Arg Arg Ser Thr Val Leu Arg Ser
    50                  55                  60

Gly Glu Val Thr Leu Val Val Thr Gln Ala Leu Ala Pro Asp Thr Pro
65                  70                  75                  80

Val Ala Arg Tyr Val Glu Arg His Gly Asp Ser Ile Ala Asp Leu Ala
                85                  90                  95

Phe Gly Cys Asp Asp Val Arg Ser Cys Phe Asp Arg Ala Val Leu Ala
            100                 105                 110

Gly Ala Glu Ala Leu Gln Ala Pro Thr Pro Ser His Arg Ala Gly Gln
        115                 120                 125

Asp Ala Trp Phe Ala Thr Val Ser Gly Phe Gly Asp Ile Arg His Thr
    130                 135                 140

Leu Val Pro Ala Ala Asp Gly Asp Gly Ala Gly Leu Leu Pro Pro Asp
145                 150                 155                 160

Arg Asp Trp Ala Leu Leu Pro Ala Ala Thr Gly Arg Thr Gly Pro Arg
                165                 170                 175

Pro Leu Leu Asp His Val Ala Val Cys Leu Glu Ser Gly Thr Leu Arg
            180                 185                 190

Ser Thr Ala Glu Phe Tyr Glu Ala Ala Phe Asp Met Pro Tyr Tyr Ser
        195                 200                 205

Ser Glu Tyr Ile Glu Val Gly Glu Gln Ala Met Asp Met Ile Met Val
    210                 215                 220

Arg Asn Ala Gly Gly Gly Ile Thr Phe Thr Leu Ile Glu Pro Asp Asp
225                 230                 235                 240

Thr Arg Val Pro Gly Gln Ile Asp Gln Phe Leu Ser Ala His Asp Gly
                245                 250                 255

Pro Gly Val Gln His Leu Ala Phe Leu Val Asp Asp Ile Val Gly Ser
            260                 265                 270

Val Arg Ser Leu Gly Asp Arg Gly Val Ala Phe Leu Arg Thr Pro Gly
        275                 280                 285

Ala Tyr Tyr Asp Leu Leu Thr Glu Arg Val Gly Ala Met Ala Asp Ala
    290                 295                 300

Ile Glu Asp Leu Arg Glu Thr Asn Val Leu Ala Asp Arg Asp Glu Trp

305                 310                 315                 320

Gly Tyr Leu Leu Gln Ile Phe Thr Arg Ser Pro Tyr Pro Arg Gly Thr
                325                 330                 335

Leu Phe Tyr Glu Tyr Ile Gln Arg Asn Gly Ala Arg Gly Phe Gly Ser
            340                 345                 350

Ser Asn Ile Lys Ala Leu Ala Glu Ala Val Glu Arg Glu Arg Glu Val
        355                 360                 365

Ala Gly Arg
    370

<210>  11
<211>  33
<212>  DNA
<213>  Artificial Sequence

<220>
<223>  Primer

<400>  11
ctatacatat gctccctcct ttccccttcc ttc                                  33

<210>  12
<211>  62
<212>  DNA
<213>  Artificial Sequence

<220>
<223>  Primer

<400>  12
atagaggatc cttatttttc gaactgcggg tggctccaag cgcttcggcc ggccacttcc      60

cg                                                                    62

<210>  13
<211>  26
<212>  DNA
<213>  Artificial Sequence

<220>
<223>  Primer

<400>  13
gatggactcc atctgggtgc gcaacg                                          26

<210>  14
<211>  26
<212>  DNA
<213>  Artificial Sequence

<220>
<223>  Primer

<400>  14
cgttgcgcac ccagatggag tccatc                                          26

<210>  15

<211>  26
<212>  DNA
<213>  Artificial Sequence

<220>
<223>  Primer

<400>  15
gatggactcc atcatggtgc gcaacg                                          26


<210>  16
<211>  26
<212>  DNA
<213>  Artificial Sequence

<220>
<223>  Primer

<400>  16
cgttgcgcac catgatggag tccatc                                          26


<210>  17
<211>  26
<212>  DNA
<213>  Artificial Sequence

<220>
<223>  Primer

<400>  17
gatggactcc atctttgtgc gcaacg                                          26


<210>  18
<211>  26
<212>  DNA
<213>  Artificial Sequence

<220>
<223>  Primer

<400>  18
cgttgcgcac aaagatggag tccatc                                          26


<210>  19
<211>  33
<212>  DNA
<213>  Artificial Sequence

<220>
<223>  Primer

<400>  19
caacatcaag gccctggccg aggccgtcga gcg                                  33


<210>  20
<211>  33
<212>  DNA
<213>  Artificial Sequence

<220>
<223>  Primer

<400>  20
cgctcgacgg cctcggccag ggccttgatg ttg                                  33

<210>  21
<211>  26
<212>  DNA
<213>  Artificial Sequence

<220>
<223>  Primer

<400>  21
gatggacatg atcgtcgtgc gcaacg                                            26


<210>  22
<211>  29
<212>  DNA
<213>  Artificial Sequence

<220>
<223>  Primer

<400>  22
tgcgcacgac gatcatgtcc atcgcctgc                                         29


<210>  23
<211>  24
<212>  DNA
<213>  Artificial Sequence

<220>
<223>  Primer

<400>  23
ccttcacgct ctttgaaccg gacg                                              24


<210>  24
<211>  24
<212>  DNA
<213>  Artificial Sequence

<220>
<223>  Primer

<400>  24
cgtccggttc aaagagcgtg aagg                                              24


<210>  25
<211>  26
<212>  DNA
<213>  Artificial Sequence

<220>
<223>  Primer

<400>  25
gatggacatg atctttgtgc gcaacg                                            26


<210>  26
<211>  29
<212>  DNA
<213>  Artificial Sequence

<220>
<223>  Primer

<400>  26

tgcgcacaaa gatcatgtcc atcgcctgc                                          29


<210>  27
<211>  26
<212>  DNA
<213>  Artificial Sequence

<220>
<223>  Primer

<400>  27
gatggacatg atcatggtgc gcaacg                                             26


<210>  28
<211>  29
<212>  DNA
<213>  Artificial Sequence

<220>
<223>  Primer

<400>  28
tgcgcaccat gatcatgtcc atcgcctgc                                          29


<210>  29
<211>  15
<212>  PRT
<213>  Artificial Sequence

<220>
<223>  Consensus sequence


<220>
<221>  MISC_FEATURE
<222>  (1)..(1)
<223>  Xaa is serine

<220>
<221>  MISC_FEATURE
<222>  (2)..(2)
<223>  Xaa is selected from the group consisting of threonine, lysine,
       isoleucine, arginine and glutamine

<220>
<221>  MISC_FEATURE
<222>  (3)..(3)
<223>  Xaa is valine or alanine

<220>
<221>  MISC_FEATURE
<222>  (4)..(4)
<223>  Xaa is selected from the group consisting of valine, methionine
       and isoleucine

<220>
<221>  MISC_FEATURE
<222>  (5)..(5)
<223>  Xaa = (Xaa)n, wherein Xaa is any amino acid residue and n is an
       integer between 100 and 150

<220>
<221>  MISC_FEATURE
<222>  (8)..(8)
<223>  Xaa is any amino acid residue

<220>

<221>  MISC_FEATURE
<222>  (9)..(9)
<223>  Xaa is any amino acid residue

<220>
<221>  MISC_FEATURE
<222>  (11)..(11)
<223>  Xaa is isoleucine or phenylalanine

<220>
<221>  MISC_FEATURE
<222>  (12)..(12)
<223>  Xaa is selected from the group consisting of lysine, glutamine,
       arginine and threonine

<220>
<221>  MISC_FEATURE
<222>  (13)..(13)
<223>  Xaa is selected from the group consisting of alanine, serine and
       glycine

<220>
<221>  MISC_FEATURE
<222>  (14)..(14)
<223>  Xaa is leucine or isoleucine

<220>
<221>  MISC_FEATURE
<222>  (15)..(15)
<223>  Xaa is tyrosine or phenylalanine

<400>  29

Xaa Xaa Xaa Xaa Xaa Phe Gly Xaa Xaa Asn Xaa Xaa Xaa Xaa Xaa
1               5                   10                  15


<210>  30
<211>  5
<212>  PRT
<213>  Artificial Sequence

<220>
<223>  Consesnsus sequence


<220>
<221>  MISC_FEATURE
<222>  (2)..(2)
<223>  Xaa = (Xaa)m, wherein Xaa is any amino acid residues and m an
       integer between 100 and 150

<220>
<221>  MISC_FEATURE
<222>  (4)..(4)
<223>  Xaa = (Xaa)o, wherein Xaa is any amino acid residues and o an
       integer between 100 and 150

<400>  30

His Xaa His Xaa Glu
1               5